(19) Europäisches Patentamt / European Patent Office / Office européen des brevets

(11) EP 3 085 753 B1

(12) **EUROPÄISCHE PATENTSCHRIFT**

(45) Veröffentlichungstag und Bekanntmachung des Hinweises auf die Patenterteilung:
**24.04.2019 Patentblatt 2019/17**

(51) Int Cl.:
***C07D 333/76*** (2006.01) ***C07D 307/91*** (2006.01)
***C09K 19/34*** (2006.01)

(21) Anmeldenummer: **16000733.2**

(22) Anmeldetag: **30.03.2016**

(54) **FLUORIERTE DIBENZOFURAN- UND DIBENZOTHIOPHENDERIVATE**

FLUORINATED DIBENZOFURANE DERIVATIVES AND DIBENZOTHIOPHENE DERIVATIVES

DERIVES DE DIBENZOTHIOPHENES ET DIBENZOFURANES FLUORES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.04.2015 DE 102015004505**

(43) Veröffentlichungstag der Anmeldung:
**26.10.2016 Patentblatt 2016/43**

(73) Patentinhaber: **Merck Patent GmbH**
**64293 Darmstadt (DE)**

(72) Erfinder:
- **LIETZAU, Lars**
  **64380 Rossdorf (DE)**
- **BROCKE, Constanze**
  **64521 Gross-Gerau (DE)**
- **REIFFENRATH, Volker**
  **64380 Rossdorf (DE)**
- **MANABE, Atsutaka**
  **64625 Bensheim (DE)**

(56) Entgegenhaltungen:
**WO-A1-02/055463** **DE-A1-102004 021 691**
**DE-A1-102005 012 585**

Bemerkungen:

Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 085 753 B1

## Beschreibung

### Gebiet der Erfindung

Die vorliegende Erfindung betrifft fluorierte Dibenzofuran- und Dibenzothiophenderivate, Verfahren zu ihrer Herstellung, Flüssigkristall-(FK-)-Medien enthaltend diese Derivate sowie elektrooptische Anzeigeelemente enthaltend diese flüssigkristallinen Medien.

### Hintergrund der Erfindung

Flüssigkristalle haben ein breites Anwendungsfeld gefunden, seitdem vor etwa 30 Jahren die ersten kommerziell anwendbaren flüssigkristallinen Verbindungen gefunden wurden. Bekannte Anwendungsgebiete für herkömmliche Mischungen sind insbesondere Anzeigen für Uhren und Taschenrechner sowie große Anzeigetafeln, wie sie in Bahnhöfen, Flughäfen und Sportarenen verwendet werden. Weitere Anwendungsgebiete sind Displays von tragbaren und stationären Computern, Navigationssystemen und Videoapplikationen. Insbesondere für die zuletzt genannten Anwendungen werden hohe Anforderungen an Schaltzeiten und den Kontrast der Abbildungen gestellt.

Die räumliche Ordnung der Moleküle in einem Flüssigkristall bewirkt, dass viele seiner Eigenschaften richtungsabhängig sind. Von Bedeutung für den Einsatz in Flüssigkristallanzeigen sind dabei insbesondere die Anisotropien im optischen, dielektrischen und elasto-mechanischen Verhalten. Je nachdem, ob die Moleküle mit ihren Längsachsen senkrecht oder parallel zu den beiden Platten eines Kondensators orientiert sind, hat dieser eine andere Kapazität; die Dielektrizitätskonstante $\varepsilon$ des flüssigkristallinen Mediums ist also für die beiden Orientierungen verschieden groß. Substanzen, deren Dielektrizitätskonstante bei senkrechter Orientierung der Moleküllängsachsen zu den Kondensatorplatten größer ist als bei paralleler Anordnung, werden als dielektrisch positiv bezeichnet. Mit anderen Worten: Ist die Dielektrizitätskonstante $\varepsilon\|$ parallel zu den Moleküllängsachsen größer als die Dielektrizitätskonstante $\varepsilon\perp$ senkrecht zu den Moleküllängsachsen, so ist die dielektrische Anisotropie $\Delta\varepsilon = \varepsilon\| - \varepsilon\perp$ größer null. Die meisten Flüssigkristalle, die in herkömmlichen Displays Verwendung finden, fallen in diese Gruppe.

Für die dielektrische Anisotropie spielen sowohl die Polarisierbarkeit des Moleküls als auch permanente Dipolmomente eine Rolle. Beim Anlegen einer Spannung an das Display richtet sich die Längsachse der Moleküle so aus, dass die größere der dielektrischen Konstanten wirksam wird. Die Stärke der Wechselwirkung mit dem elektrischen Feld hängt dabei von der Differenz der beiden Konstanten ab.

Bei den in herkömmlichen Flüssigkristallanzeigen verwendeten flüssigkristallinen Molekülen ist das entlang der Moleküllängsachse orientierte Dipolmoment größer als das senkrecht zur Moleküllängsachse orientierte Dipolmoment.

Mit Flüssigkristallen, bei denen das größere Dipolmoment parallel zur Längsachse des Moleküls orientiert ist, sind bereits sehr leistungsfähige Displays entwickelt worden. Dabei kommen meist Mischungen von 5 bis 20 Komponenten zum Einsatz, um einen ausreichend breiten Temperaturbereich der Mesophase sowie kurze Schaltzeiten und niedrige Schwellenspannungen anzustreben. Schwierigkeiten bereitet jedoch noch die starke Blickwinkelabhängigkeit bei Flüssigkristallanzeigen, wie sie beispielsweise für Laptops verwendet werden. Die beste Abbildungsqualität lässt sich erreichen, wenn die Fläche des Displays senkrecht zur Blickrichtung des Betrachters steht. Wird das Display relativ zur Betrachtungsrichtung gekippt, verschlechtert sich die Abbildungsqualität unter Umständen drastisch. Für einen höheren Komfort ist man bemüht, den Winkel, um den das Display von der Blickrichtung eines Betrachters ohne wesentliche Minderung der Abbildungsqualität verkippt werden kann, möglichst groß zu gestalten. In jüngerer Zeit sind Versuche unternommen worden, zur Verbesserung der Blickwinkelabhängigkeit flüssigkristalline Verbindungen einzusetzen, deren Dipolmoment senkrecht zur Moleküllängsachse größer ist als parallel zur Längsachse des Moleküls. Die dielektrische Anisotropie $\Delta\varepsilon$ ist in diesem Fall negativ. Im feldfreien Zustand werden diese Moleküle mit ihrer Längsachse senkrecht zur Glasfläche des Displays orientiert. Durch Anlegen eines elektrischen Feldes orientieren sie sich mehr oder weniger parallel zu den Glasflächen. Auf diese Weise konnte eine Verbesserung der Blickwinkelabhängigkeit erreicht werden. Derartige Displays werden als VA-TFT-Displays bezeichnet (abgeleitet aus dem Englischen: "vertically aligned").

Die Entwicklung auf dem Gebiet der flüssigkristallinen Materialien ist bei weitem noch nicht abgeschlossen. Zur Verbesserung der Eigenschaften flüssigkristalliner Anzeigeelemente ist man ständig bemüht, neue Verbindungen zu entwickeln, die eine Optimierung derartiger Displays ermöglichen.

Eine Aufgabe der vorliegenden Erfindung ist es, Verbindungen mit vorteilhaften Eigenschaften für den Einsatz in flüssigkristallinen Medien zur Verfügung zu stellen. Insbesondere sollten sie über eine negative dielektrische Anisotropie verfügen, was sie besonders geeignet macht für den Einsatz in flüssigkristallinen Medien für VA-Displays. Unabhängig von der dem Displaytyp entsprechenden dielektrischen Anisotropie sind Verbindungen gewünscht, die eine günstige Kombination der anwendungstechnischen Parameter aufweisen. Unter diesen gleichzeitig zu optimierenden Parametern sind vor allem zu nennen ein hoher Klärpunkt, eine geringe Rotationsviskosität, eine optische Anisotropie

im Anwendungsintervall, sowie die Eigenschaften, die zur Erzielung von Mischungen mit den gewünschten flüssigkristallinen Phasen über einen breiten Temperaturbereich dienen (niedriger Schmelzpunkt, gute Mischbarkeit mit anderen flüssigkristallinen Komponenten der gewünschten Art).

Weitere FK-Anzeigemodi, die besonders auch für kleine und mittelgroße FK-Anzeigen zur Anwendung in tragbaren Geräten wie beispielsweise Tablet-PC oder sog. Smart-Phones zu Anwendung kommen, sind der IPS- und der FFS-Modus (engl. fringe field switching), in denen FK-Medien mit positiver dielektrischer Anisotropie zum Einsatz kommen. Aus dem Stand der Technik ist bekannt, dass sich die Eigenschaften einer Flüssigkristallanzeige vom FFS-Typ verbessern lassen, indem man zu hochpolaren FK-Medien mit positiver dielektrischer Anisotropie Flüssigkristallmaterialien mit negativer dielektrischer Anisotropie zusetzt, wodurch die Dielektrizitätskonstante $\varepsilon\perp$ senkrecht zu den Moleküllängsachsen der FK-Mischung erhöht wird (s. EP 2 628 779 A2). Folglich muss die hohe negative dielektrische Anisotropie der zugemischten Substanzen wieder durch einen höheren Anteil an Materialien mit positiver dielektrischer Anisotropie ausgeglichen werden, um die zum Schalten erforderliche Polarität der Mischung herzustellen. Es besteht daher ein Bedarf an FK-Mischungskomponenten, die zwar ein hohes $\varepsilon\perp$ besitzen, aber aufgrund eines relativ niedrigen $\Delta\varepsilon$ die Polarität einer FK-Mischung mit pos. $\Delta\varepsilon$ weniger stark herabsetzen.

Eine weitere Aufgabe der vorliegenden Erfindung ist es daher, Verbindungen bereitzustellen, die neben den bereits erwähnten vorteilhaften anwendungstechnischen Eigenschaften besonders hohe Werte für $\varepsilon\perp$ bei dem Betrage nach relativ geringen Werten für $\Delta\varepsilon$ aufweisen. In anderen Worten muss das Verhältnis von $\varepsilon\perp$ zu $|\Delta\varepsilon|$ möglichst groß sein.

Aus dem Stand der Technik sind VA-Materialien bekannt, die vom Dibenzofuran oder vom Dibenzothiophen abgeleitet sind.

In WO 02/055463 sind Verbindungen der Formel

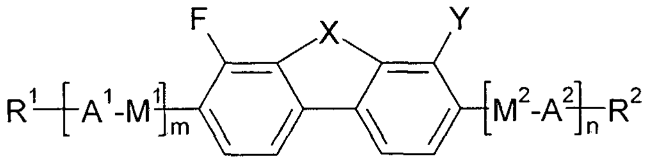

offenbart, worin X u.a. O oder S, Y F, $R^1$ und $R^2$ Alkyl oder Alkoxy bedeuten können und die übrigen Parameter die dort angegebene Bedeutung haben. Die dort beschriebenen Verbindungen besitzen neg. dielektrische Anisotropie, sind allerdings für ferroelektrische FK-Mischungen entwickelt worden und es werden keine Werte für die dielektrischen Anisotropien der Einzelsubstanzen beschrieben.

Ähnliche Verbindungen sind in DE 10 2004 021 691 A1 offengelegt, worin die Gruppe X polare Reste wie F oder $-CF_3$ generisch umfasst, bevorzugt aber H bedeutet.

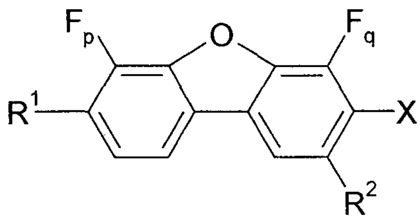

In DE 10 2005 012 585 A1 sind u.a. Dibenzofuran- und Dibenzothiophenverbindungen der allgemeinen Formel

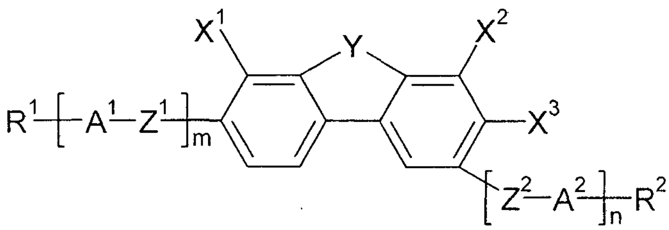

beschrieben, worin unter anderem Y O oder S, der Rest $R^1$ Alkoxy, $R^2$ H, m und n 0 und die Reste $X^1$, $X^2$ und $X^3$ F bedeuten können, wie z.B. in Verbindung des Beispiels Nr. 97

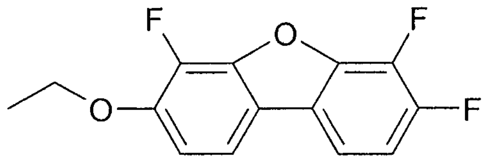
.

Die Verbindungen sind hochpolar und als Komponenten für Flüssigkristallmischungen für VA-Anzeigen entwickelt worden.

Zusammenfassung der Erfindung

Überraschend wurde gefunden, dass eine oder mehrere der oben beschriebenen Aufgaben gelöst werden können durch Verbindungen der Formel I

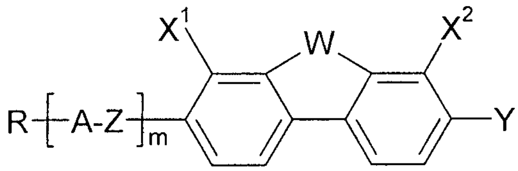
I

worin

W O oder S,

Y F, Cl, $CF_3$, $OCF_3$, $OCF_2H$, mit der Maßgabe, dass wenn W O bedeutet Y nicht F sein kann,

$X^1$, $X^2$ H oder F, mit der Maßgabe, dass mindestens ein Rest $X^1$ oder $X^2$ F bedeutet,

R einen Alkyl- oder Alkoxyrest mit 1 bis 15 C-Atomen, wobei in diesen Resten auch eine oder mehrere $CH_2$-Gruppen jeweils unabhängig voneinander durch -C≡C-, $-CF_2O-$, $-OCF_2-$, -CH=CH-,

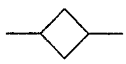 , 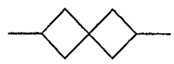 ,

-O-, -CO-O-, oder -O-CO- so ersetzt sein können, dass O-Atome nicht direkt miteinander verknüpft sind, und worin auch ein oder mehrere H-Atome durch Halogen ersetzt sein können,

A bei jedem Auftreten gleich oder verschieden einen Rest ausgewählt aus folgenden Gruppen:

a) 1,4-Phenylen worin auch eine oder zwei CH-Gruppen durch N ersetzt sein können und worin auch ein oder mehrere H-Atome durch eine Gruppe L ersetzt sein können,

b) der Gruppe bestehend aus trans-1,4-Cyclohexylen und 1,4-Cyclohexenylen , worin auch eine oder mehrere nicht benachbarte $CH_2$-Gruppen durch -O- und/oder -S- ersetzt sein können und worin auch ein oder mehrere H-Atome durch F oder Cl ersetzt sein können, und

c) der Gruppe bestehend aus Tetrahydropyran-2,5-diyl, 1,3-Dioxan-2,5-diyl, Tetrahydrofuran-2,5-diyl, Cyclobutan-1,3-diyl, Piperidin-1,4-diyl, Thiophen-2,5-diyl und Selenophen-2,5-diyl, welche auch ein oder mehrfach durch ein Gruppe L substituiert sein können,

L bei jedem Auftreten gleich oder verschieden F, Cl, CN, SCN, $SF_5$ oder geradkettiges oder verzweigtes, jeweils optional fluoriertes, Alkyl, Alkoxy, Alkylcarbonyl, Alkoxycarbonyl, Alkylcarbonyloxy oder Alkoxycarbonyloxy mit 1 bis 12 C-Atomen, und

Z bei jedem Auftreten gleich oder verschieden eine Einfachbindung, $-CF_2O-$, $-OCF_2-$, $-CH_2O-$, $-OCH_2-$, -(CO)O-, -O(CO)-, $-(CH_2)_4-$, $-CH_2CH_2-$, $-CF_2-CF_2-$, $-CF_2-CH_2-$, $-CH_2-CF_2$, -CH=CH-, -CF=CF- , -CF=CH-, -CH=CF-, $-(CH_2)_3O-$, $-O(CH_2)_3-$, -C≡C-, -O-, $-CH_2-$, $-(CH_2)_3-$ oder $-CF_2-$,

m 0,1 oder 2,

bedeuten.

Die Verbindungen besitzen ein negatives $\Delta\varepsilon$ und eignen sich daher insbesondere für eine Verwendung in VA-TFT-Displays und ganz besonders als Additiv in IPS- oder FFS-Displays. Vorzugsweise besitzen die erfindungsgemäßen Verbindungen ein $\Delta\varepsilon$ zwischen 0 und -8, besonders bevorzugt zwischen -2 und -4, bei Werten für $\varepsilon\perp$ von vorzugsweise > 10, besonders bevorzugt > 15. Bevorzugt ist das Verhältnis $\varepsilon\perp /|\Delta\varepsilon|$ > 3, besonders bevorzugt >5. Sie zeigen eine gute Mischbarkeit mit den üblichen, in Flüssigkristallmischungen für Displays verwendeten Substanzen, d. h. sie besitzen eine gute Löslichkeit darin. Die Rotationsviskositäten der Verbindungen und der resultierenden flüssigkristallinen Mischungen sind vorteilhaft klein.

Auch die weiteren physikalischen, physikochemischen beziehungsweise elektrooptischen Parameter der erfindungsgemäßen Verbindungen sind für den Einsatz der Verbindungen in flüssigkristallinen Medien von Vorteil. Die flüssigkristallinen Medien, die diese Verbindungen enthalten, weisen insbesondere eine ausreichende Breite der nematischen Phase und eine gute Tieftemperatur- und Langzeitstabilität sowie ausreichend hohe Klärpunkte auf. Die niedrigen Schmelzpunkte der erfindungsgemäßen Verbindungen geben einen Hinweis auf das vorteilhafte Mischungsverhalten. Ferner weisen die erfindungsgemäßen Verbindungen der Formel I insbesondere für die Verwendung in VA-TFT-Displays geeignete Werte der optischen Anisotropie $\Delta n$ auf. Bevorzugt besitzen die erfindungsgemäßen Verbindungen ein $\Delta n$ von größer als 0,15 und kleiner als 0,25.

<u>Detaillierte Beschreibung der Erfindung</u>

Nachfolgend wird die beste Ausführungsform der vorliegenden Erfindung detailliert beschrieben.

Bevorzugt bedeutet R einen Alkoxyrest, Alkylrest oder Alkenylrest mit 1 bis 7 bzw. 2 bis 7 Kohlenstoffatomen. Besonders bevorzugt ist R in der allgemeinen Formel I ein Alkoxyrest oder Alkylrest mit 2 bis 7 C-Atomen. Für den Fall, dass m = 0 ist, bedeutet R vorzugsweise eine Alkoxygruppe mit 1-7 C-Atomen, besonders bevorzugt mit 2 bis 5 C-Atomen.

Für den Fall, dass m = 1 oder 2 ist, bedeutet R vorzugsweise eine Alkyl-, Alkoxy- oder Alkenylgruppe, besonders bevorzugt eine Alkyl- oder Alkenylgruppe mit 1-7 C-Atomen, besonders bevorzugt mit 2 bis 5 C-Atomen.

Sofern R einen Alkylrest darstellt, ist dieser geradkettig oder verzweigt. Vorzugsweise ist R geradkettig, und hat soweit nicht anders angegeben 1, 2, 3, 4, 5, 6 oder 7 C-Atome und ist demnach vorzugsweise Methyl, Ethyl, Propyl, Butyl, Pentyl, Hexyl oder Heptyl.

Sofern R einen Alkoxyrest darstellt, sind dieser geradkettig oder verzweigt. Vorzugsweise ist R geradkettig, und hat soweit nicht anders angegeben 1, 2, 3, 4, 5, 6 oder 7 C-Atome und ist demnach vorzugsweise Methoxy, Ethoxy, Propoxy, Butoxy, Pentoxy, Hexoxy oder Heptoxy.

R in Formel I kann ferner ein Alkenylrest mit 2 bis 15 C-Atomen sein, der geradkettig oder verzweigt ist und wenigstens eine C-C-Doppelbindung aufweist. Vorzugsweise ist er geradkettig und hat 2 bis 7 C-Atome. Er ist demnach vorzugsweise Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl. Sind die beiden C-Atome der C-C-Doppelbindung substituiert, kann der Alkenylrest als E- und/oder Z-Isomer (trans/cis) vorliegen. Im Allgemeinen sind die jeweiligen E-Isomere bevorzugt. Unter den Alkenylresten sind besonders bevorzugt Prop-2-enyl, 2- oder But-3-enyl, und 3- oder Pent-4-enyl.

R in Formel I kann auch ein Alkinylrest mit 2 bis 15 C-Atomen sein, der geradkettig oder verzweigt ist und wenigstens eine C-C-Dreifachbindung aufweist. Bevorzugt ist 1- oder 2 Propinyl und 1-, 2- oder 3- Propinyl.

Die Gruppe A bedeutet bei jedem Auftreten gleich oder verscheiden bevorzugt eine disubstituierte ringförmige Gruppe ausgewählt aus den Formeln

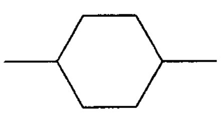 , 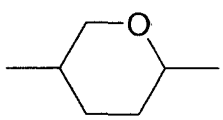 , 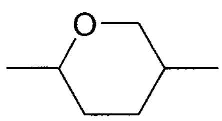 , 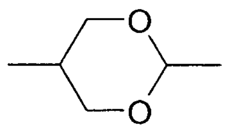 ,

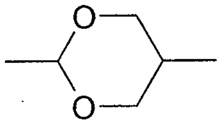 , 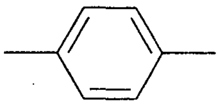 , 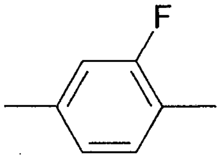 , 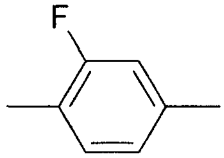

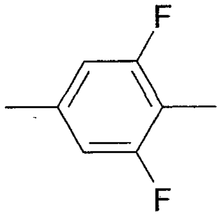 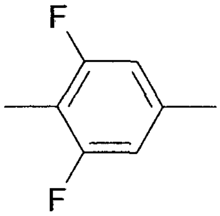 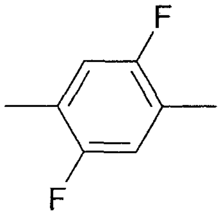 oder 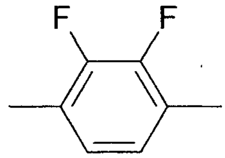 ,

insbesondere

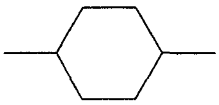 , 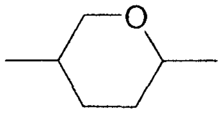 , 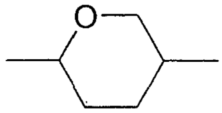 ,

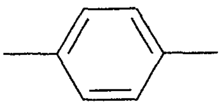 , 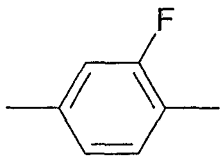 , 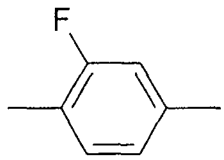 oder

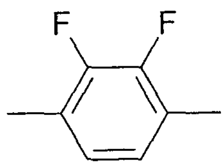 .

Die Gruppe Z bedeutet bevorzugt eine Einfachbindung, $-CH_2O-$, $-CF_2O-$ oder $-OCF_2-$, besonders bevorzugt $-CH_2O-$.

Die Gruppe W bedeutet bevorzugt O.

Für die Gruppen $X^1$ und $X^2$ ist es bevorzugt, dass beide F bedeuten.

Die Gruppe Y bedeutet bevorzugt $-CF_3$ oder $-OCF_3$, besonders bevorzugt $-OCF_3$.

Die Gruppe L bedeutet bevorzugt F, Cl, $-CF_3$ oder eine Alkyl- oder Alkoxygruppe mit 1, 2 oder 3 Kohlenstoffatomen, besonders bevorzugt F.

Der Parameter m besitzt bevorzugt einen Wert von 0 oder 1,

Besonders bevorzugt bedeuten m 0 und R eine Alkoxygruppe mit 1 bis 7 Kohlenstoffatomen.

Halogen bedeutet im Zusammenhang der vorliegenden Erfindung Fluor, Chlor, Brom oder Iod, insbesondere Fluor oder Chlor.

Im Zusammenhang mit der vorliegenden Erfindung bedeutet der Ausdruck "Alkyl" - sofern er nicht an anderer Stelle dieser Beschreibung oder in den Ansprüchen abweichend definiert ist - einen geradkettigen oder verzweigten, gesättigten, aliphatischen Kohlenwasserstoffrest mit 1 bis 15 Kohlenstoffatomen.

Besonders bevorzugt sind erfindungsgemäße Verbindungen der Formel I ausgewählt aus den Unterformeln Ia bis Id,

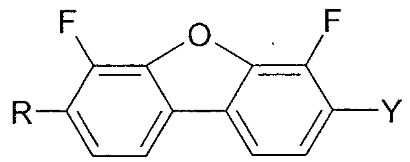

Ia

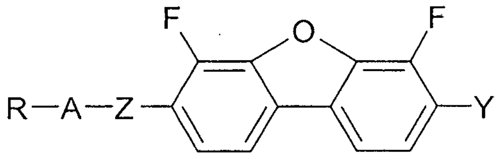

Ib

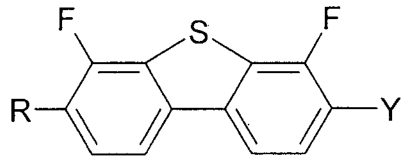

Ic

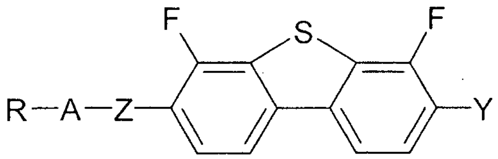

Id

wobei die Reste R, A, Z und Y die oben angegebenen Bedeutungen haben.

Bevorzugte Verbindungen der Formeln Ia, Ib, Ic und Id sind die Verbindungen der Formeln la und Ib mit den bevorzugten Unterformeln Ia-1 und Ia-2,

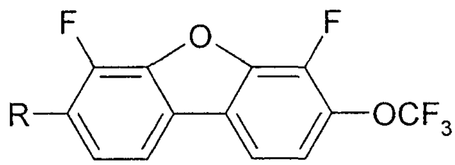

Ia-1

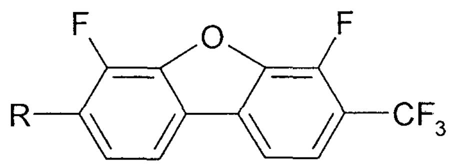

Ia-2

worin R eine geradkettige Alkoxygruppe mit 1 bis 5 C-Atomen bedeutet,
sowie Ib-1 und 1b-2,

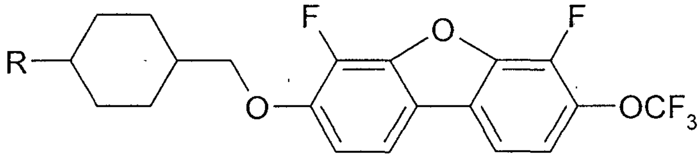

Ib-1

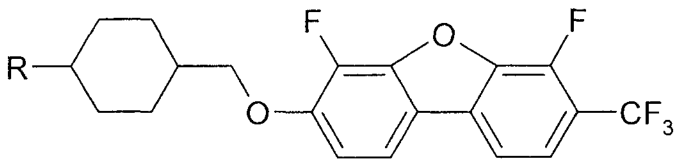

Ib-2

worin R eine geradkettige Alkyl- oder Alkenylgruppe mit 2 bis 5 C-Atomen bedeutet.

Ganz besonders bevorzugt sind Verbindungen der Formeln Ia-1-1 bis 1a-1-8 und Ib-1-1 bis Ib-1-5,

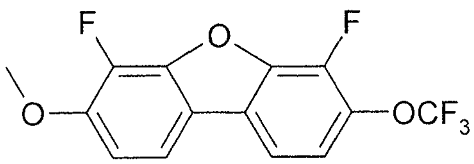

Ia-1-1

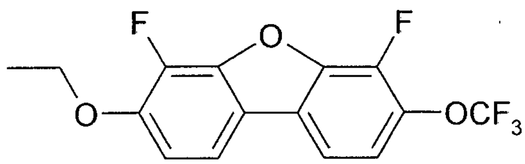

Ia-1-2

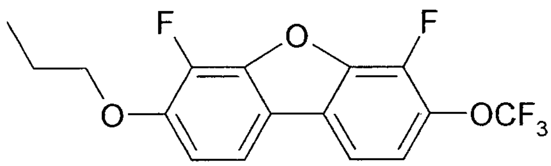

Ia-1-3

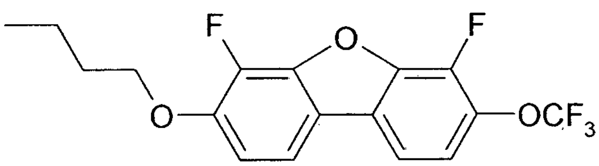

Ia-1-4

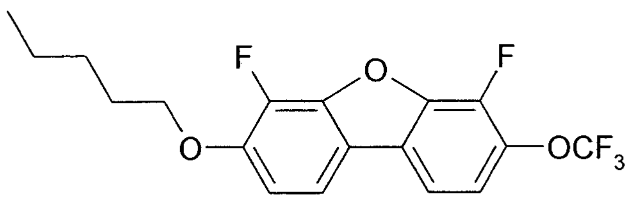

Ia-1-5

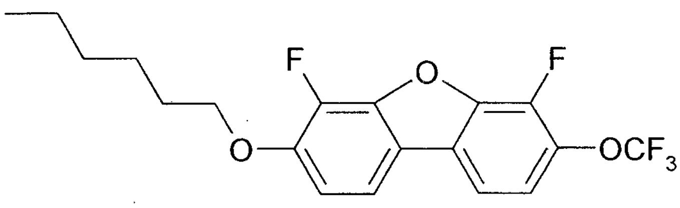

Ia-1-6

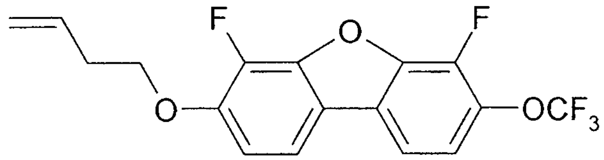

Ia-1-7

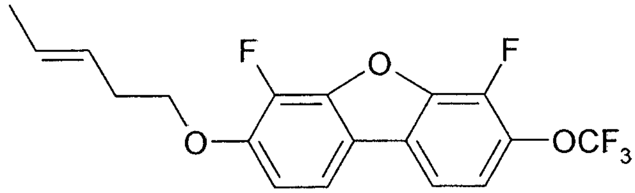

Ia-1-8

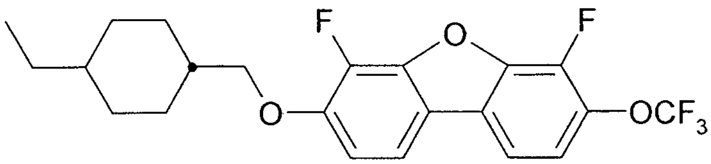

Ib-1-1

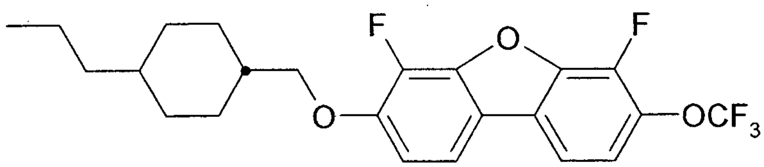

Ib-1-2

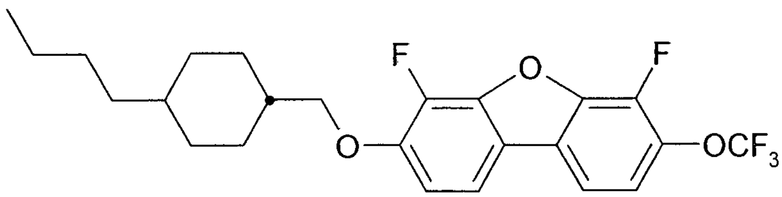

Ib-1-3

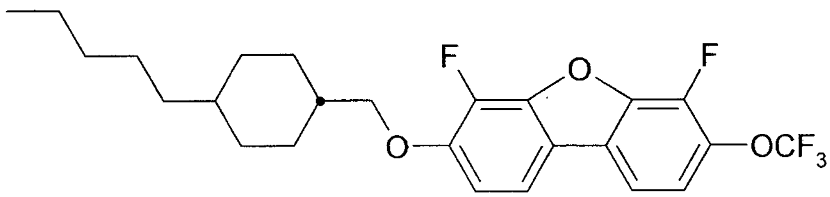

Ib-1-4

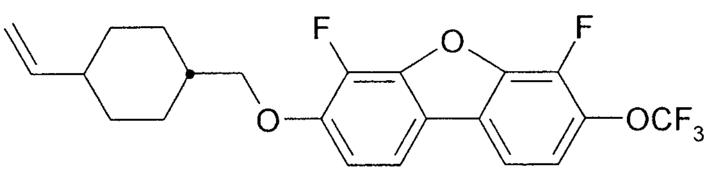

Ib-1-5

Sofern Reste oder Substituenten der erfindungsgemäßen Verbindungen oder die erfindungsgemäßen Verbindungen selbst als optisch aktive oder stereoisomere Reste, Substituenten oder Verbindungen vorliegen, weil sie beispielsweise ein asymmetrisches Zentrum aufweisen, so sind diese von der vorliegenden Erfindung mit umfasst. Dabei ist es selbstverständlich, dass die erfindungsgemäßen Verbindungen der allgemeinen Formel I in isomerenreiner Form, zum Beispiel als reine Enantiomeren, Diastereomeren, E- beziehungsweise Z-Isomeren, trans- oder cis-Isomeren, oder als Gemisch mehrerer Isomeren in jedem beliebigen Verhältnis, zum Beispiel als Racemat, E-/Z-Isomerengemisch oder

als cis/trans-Isomerengemisch, vorliegen können.

Der 1,4-substituierte Cyclohexylring der Formel

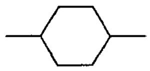

oder -Cyc- ist in den offenbarten Verbindungen für flüssigkristalline Medien bevorzugt trans-konfiguriert, d.h. die beiden Substituenten befindend sich in der thermodynamisch bevorzugten Sesselkonformation beide in äquatorialer Position.

Die Verbindungen der allgemeinen Formel I können nach an sich bekannten Methoden dargestellt werden, wie sie in der Literatur (z.B. in den Standardwerken wie Houben-Weyl, Methoden der organischen Chemie, Georg-Thieme-Verlag, Stuttgart) beschrieben sind und zwar unter Reaktionsbedingungen, die für die genannten Umsetzungen bekannt und geeignet sind. Dabei kann man von an sich bekannten, hier nicht näher erwähnten Varianten Gebrauch machen.

Die Ausgangsstoffe können gegebenenfalls auch in situ gebildet werden, derart, dass man sie aus dem Reaktionsgemisch nicht isoliert, sondern sofort weiter zu den Verbindungen der allgemeinen Formel I umsetzt.

Die Synthesen erfindungsgemäßer Verbindungen der allgemeinen Formel I werden in den Beispielen exemplarisch beschrieben. Die

Ausgangssubstanzen sind nach allgemein zugänglichen Literaturvorschriften oder käuflich zu erhalten.

Besonders geeignete Synthesewege zu den erfindungsgemäßen Verbindungen werden im Folgenden anhand Schema 1 erläutert.

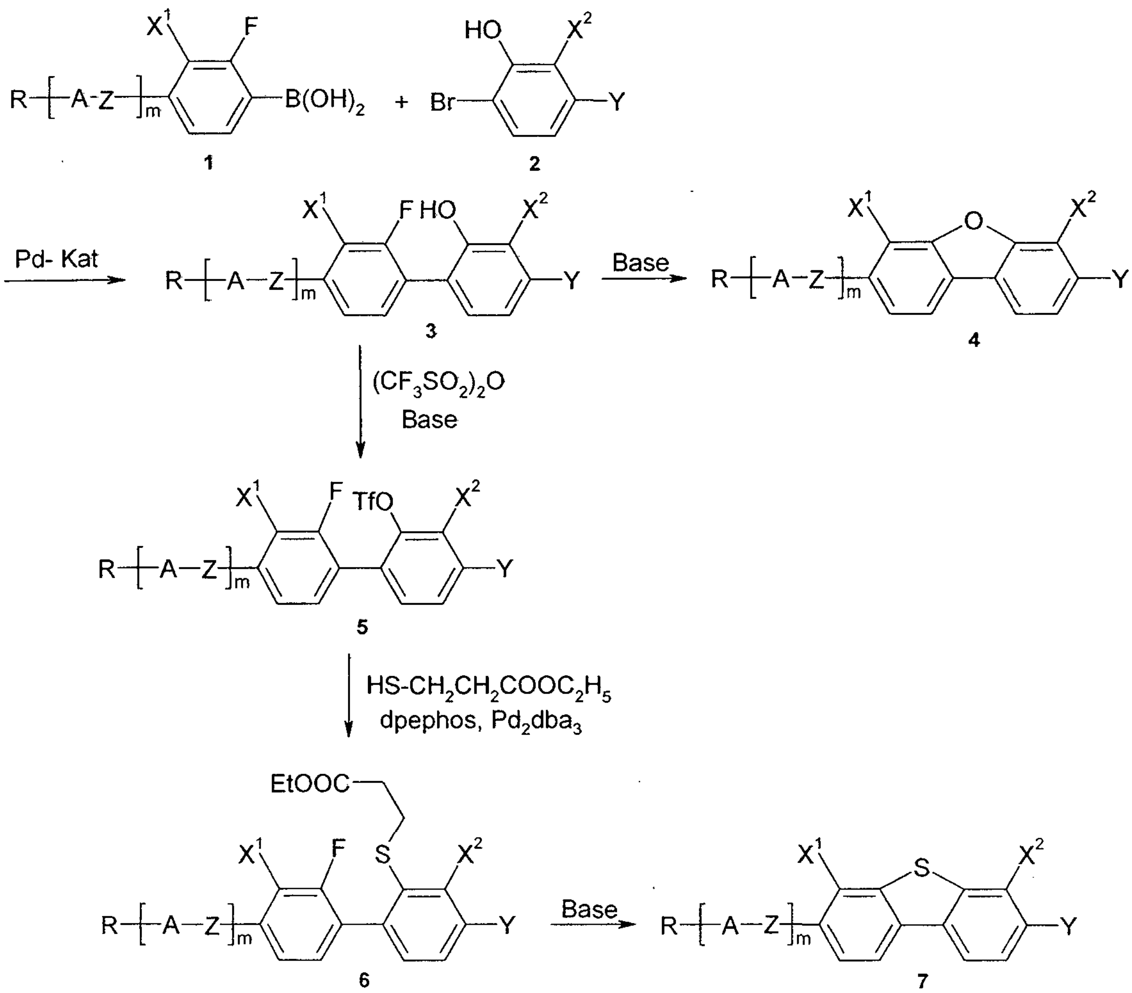


**Schema 1**. Synthese der Verbindungen der Formel I. Die Reste R, A, Z, $X^1$, $X^2$, Y sowie der Zähler m haben die für Formel I angegebene Bedeutung.

Schema 1 ist nur als beispielhaft aufzufassen. Der Fachmann kann entsprechende Variationen der vorgestellten Synthesen vornehmen, sowie auch andere geeignete Synthesewege beschreiten, um Verbindungen der Formel I zu erhalten.

Gemäß der zuvor dargestellten Synthese umfasst die vorliegende Erfindung in einer Ausführungsform auch ein oder mehrere Verfahren zur Herstellung von Verbindungen der Formel I.

Die Erfindung umfasst somit ein Verfahren zur Herstellung von Verbindungen der Formel I, das dadurch gekennzeichnet ist, dass es einen Verfahrensschritt umfasst, in dem eine Verbindung der Formel II in Gegenwart einer Base zu Verbindungen der Formel I umgesetzt wird, wie in Schema 2 gezeigt ist und worin R, A, Z, $X^1$, $X^2$, W und m die oben angegebene Bedeutung haben und G -OH, -SH oder SG' bedeutet und G' eine basenlabile Schutzgruppe für Thiole bedeutet. Bevorzugte Schutzgruppen sind Acetyl, Dimethylaminocarbonyl, 2-Tetrahydropyranyl, Ethoxycarbonylethyl, tert.-Butyl, Methyl, besonders bevorzugt Ethoxycarbonylethyl.

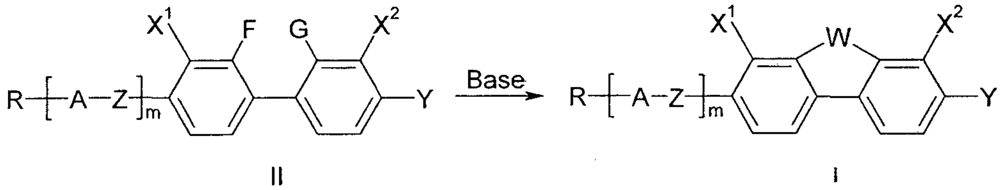


Schema 2. Verfahren zur Herstellung der Verbindungen der Formel I.

Das Verfahren und die anschließende Aufarbeitung des Reaktionsgemisches kann grundsätzlich als Batch-Reaktion oder in kontinuierlicher Reaktionsweise durchgeführt werden. Die kontinuierliche Reaktionsweise umfasst z. B. die Reaktion in einem kontinuierlichen Rührkesselreaktor, einer Rührkesselkaskade, einem Schlaufen- oder Querstromreaktor, einem Strömungsrohr oder in einem Mikroreaktor. Die Aufarbeitung der Reaktionsgemische erfolgt wahlweise, je nach Bedarf, durch Filtration über feste Phasen, Chromatographie, Separation zwischen nicht mischbaren Phasen (z. B. Extraktion), Adsorption an festen Trägern, Abdestillieren von Lösungsmitteln und/oder azeotropen Gemischen, selektive Destillation, Sublimation, Kristallisation, Cokristallisation oder durch Nanofiltration an Membranen.

Wie bereits erwähnt, können die Verbindungen der allgemeinen Formel in flüssigkristallinen Medien verwendet werden. Gegenstand der vorliegenden Erfindung ist daher auch ein flüssigkristallines Medium mit mindestens zwei flüssigkristallinen Verbindungen, enthaltend mindestens eine Verbindung der allgemeinen Formel I.

Gegenstand der vorliegenden Erfindung sind auch flüssigkristalline Medien enthaltend neben einer oder mehreren erfindungsgemäßen Verbindungen der Formel I als weitere Bestandteile 2 bis 40, vorzugsweise 4 bis 30 Komponenten. Besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, 1,3-Dioxane, 2,5-Tetrahydro-pyrane, Phenyl- oder Cyclohexylbenzoate, Cyclohexancarbonsäurephenyl- oder-cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Biscyclohexylbenzole, 4',4'-Biscyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenylcyclohexyl)ethane, 1-Cyclohexyl-2-biphenylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch einfach oder mehrfach fluoriert sein.

Die wichtigsten als weitere Bestandteile erfindungsgemäßer Medien in Frage kommenden Verbindungen lassen sich durch die Formeln (II), (III), (IV), (V) und (VI) charakterisieren:

R'-L-E-R" (II)

R'-L-COO-E-R" (III)

R'-L-OOC-E-R" (IV)

R'-L-$CH_2CH_2$-E-R" (V)

R'-L-$CF_2O$-E-R" (VI)

In den Formeln (II), (III), (IV), (V) und (VI) bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -Thp-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl, Thp Tetrahydropyran-2,5-diyl und G 2-(trans-1,4-Cyclohexyl)-ethyl, Pyrimidin-2,5-diyl, Pyridin-2,5-diyl, 1,3-Dioxan-2,5-diyl oder Tetrahydropyran-2,5-diyl bedeuten.

Vorzugsweise ist einer der Reste L und E Cyc oder Phe. E ist vorzugsweise Cyc, Phe oder Phe-Cyc. Vorzugsweise enthalten die erfindungsgemäßen Medien eine oder mehrere Komponenten ausgewählt aus den Verbindungen der Formeln (II), (III), (IV), (V) und (VI), worin L und E ausgewählt sind aus der Gruppe Cyc und Phe und gleichzeitig

eine oder mehrere Komponenten ausgewählt aus den Verbindungen der Formeln (II), (III), (IV), (V) und (VI), worin einer der Reste L und E ausgewählt ist aus der Gruppe Cyc und Phe und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln (II), (III), (IV), (V) und (VI), worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln (II), (III), (IV), (V) und (VI) jeweils unabhängig voneinander Alkyl, Alkenyl, Alkoxy, Alkoxyalkyl (Oxaalkyl), Alkenyloxy oder Alkanoyloxy mit bis zu 8 C-Atomen. Im folgenden wird diese kleinere Untergruppe Gruppe A genannt und die Verbindungen werden mit den Teilformeln (IIa), (lila), (IVa), (Va) und (VIa) bezeichnet. Bei den meisten dieser Verbindungen sind R' und R" voneinander verschieden, wobei einer dieser Reste meist Alkyl, Alkenyl, Alkoxy oder Alkoxyalkyl (Oxaalkyl) ist.

In einer anderen als Gruppe B bezeichneten kleineren Untergruppe der Verbindungen der Formeln (II), (III), (IV), (V) und (VI) bedeutet R" -F, -Cl, -NCS oder $-(O)_iCH_{3-k}F_k$, wobei i 0 oder 1 und k 1, 2 oder 3 ist. Die Verbindungen, in denen R" diese Bedeutung hat, werden mit den Teilformeln (IIb), (IIIb), (IVb), (Vb) und (VIb) bezeichnet. Besonders bevorzugt sind solche Verbindungen der Teilformeln (IIb), (IIIb), (IVb), (Vb) und (VIb), in denen R" die Bedeutung -F, -Cl, -NCS, $-CF_3$, $-OCHF_2$ oder - $OCF_3$ hat.

In den Verbindungen der Gruppe B, die mit den Teilformeln (IIb), (IIIb), (IVb), (Vb) und (VIb) bezeichnet werden, hat R' die bei den Verbindungen der Teilformeln (IIa) bis (VIa) angegebene Bedeutung und ist vorzugsweise Alkyl, Alkenyl, Alkoxy oder Alkoxyalkyl (Oxaalkyl).

In einer weiteren kleineren Untergruppe der Verbindungen der Formeln (II), (III), (IV), (V) und (VI) bedeutet R" -CN. Diese Untergruppe wird im Folgenden als Gruppe C bezeichnet und die Verbindungen dieser Untergruppe werden entsprechend mit Teilformeln (IIc), (IIIc), (IVc), (Vc) und (VIc) beschrieben. In den Verbindungen der Teilformeln (IIc), (IIIc), (IVc), (Vc) und (VIc) hat R' die bei den Verbindungen der Teilformeln (IIa) bis (VIa) angegebene Bedeutung und ist vorzugsweise Alkyl, Alkenyl, Alkoxy oder Alkoxyalkyl (Oxaalkyl).

Neben den bevorzugten Verbindungen der Gruppen A, B und C sind auch andere Verbindungen der Formeln (II), (III), (IV), (V) und (VI) mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben den erfindungsgemäßen Verbindungen der allgemeinen Formel I vorzugsweise eine oder mehrere Verbindungen aus den Gruppen A, B und/oder C. Die Massenanteile der Verbindungen aus diesen Gruppen an den erfindungsgemäßen Medien betragen:

Gruppe A:
0 bis 90 %, vorzugsweise 20 bis 90 %, insbesondere 30 bis 90 %.
Gruppe B:
0 bis 80 %, vorzugsweise 10 bis 80 %, insbesondere 10 bis 70 %.
Gruppe C:
0 bis 80 %, vorzugsweise 5 bis 80 %, insbesondere 5 bis 50 %.

Die erfindungsgemäßen Medien enthalten vorzugsweise 1 bis 40 %, besonders bevorzugt 5 bis 30 % an den erfindungsgemäßen Verbindungen der Formel I. Die Medien enthalten vorzugsweise eine, zwei, drei, vier oder fünf erfindungsgemäße Verbindungen der Formel I.

Die Herstellung der erfindungsgemäßen Medien erfolgt in an sich üblicher Weise. In der Regel werden die Komponenten ineinander gelöst, vorzugsweise bei erhöhter Temperatur. Durch geeignete Zusätze können die flüssigkristallinen Phasen der vorliegenden Erfindung so modifiziert werden, dass sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die Verbindungen der Formel I eignen sich wegen ihres negativen $\Delta\varepsilon$ für die Verwendung in VA-TFT-Displays.

Insbesondere eignen sich die Verbindungen der Formel I wegen ihres hohen Wertes für $\varepsilon_\perp$ für die Verwendung in FFS-TFT-Displays.

Gegenstand der vorliegenden Erfindung sind daher auch elektrooptische Flüssigkristallanzeigeelemente, enthaltend ein erfindungsgemäßes flüssigkristallines Medium.

Weitere Kombinationen der Ausführungsformen und Varianten der Erfindung gemäß der Beschreibung ergeben sich aus den Ansprüchen.

Die Erfindung wird im Folgenden anhand von Ausführungsbeispielen näher erläutert, ohne dadurch jedoch eingeschränkt werden zu sollen. Der Fachmann wird in der Lage sein, den Beispielen Details zur Durchführung zu entnehmen, die in der allgemeinen Beschreibung nicht im Einzelnen aufgeführt sind, sie nach allgemeinen Fachkenntnissen zu verallgemeinern und auf seine spezielle Problemstellung anzuwenden.

Neben den üblichen und wohlbekannten Abkürzungen werden folgende Abkürzungen verwendet:

K: Kristalline Phase; N: Nematische Phase; Sm: Smektische Phase;
I: Isotrope Phase. Die Zahlen zwischen diesen Symbolen geben die Übergangstemperaturen der betreffenden Substanz wieder.

Temperaturangaben sind, soweit nichts anderes angegeben, in °C.

Die Bestimmung physikalischer, physikochemischer beziehungsweise elektrooptischer Parameter erfolgt nach allgemein bekannten Verfahren, wie sie unter anderem beschrieben sind in der Broschüre "Merck Liquid Crystals - Licristal® - Physical Properties of Liquid Crystals - Description of the Measurements Methods", 1998, Merck KGaA, Darmstadt.

Vor- und nachstehend bedeutet $\Delta n$ die optische Anisotropie (589 nm, 20 °C) und $\Delta\varepsilon$ die dielektrische Anisotropie (1 kHz, 20 °C). Die dielektrische Anisotropie $\Delta\varepsilon$ wird bei 20°C und 1 kHz bestimmt. Die optische Anisotropie $\Delta n$ wird bei 20°C und einer Wellenlänge von 589,3 nm bestimmt.

Die $\Delta\varepsilon$- und $\Delta n$-Werte, sowie die Rotationsviskosität ($\gamma_1$) der erfindungsgemäßen Verbindungen werden durch lineare Extrapolation aus flüssigkristallinen Mischungen erhalten, die zu 5 bis 10 % aus der jeweiligen erfindungsgemäßen Verbindung und zu 90-95 % aus der kommerziell erhältlichen Flüssigkristallmischung ZLI-2857 (für $\Delta\varepsilon$) bzw. ZLI-4792 (für $\Delta n$, $\gamma 1$) bestehen (Mischungen Fa. Merck KGaA, Darmstadt).

Vor- und nachstehend bedeuten die Abkürzungen:

| | |
|---|---|
| DMAP | 4-(N,N-Dimethylamino)pyridin |
| DMPU | 1,3-Dimethyl-3,4,5,6-tetrahydro-2(1H)-pyrimidinon |
| dpephos | Bis[2-(diphenylphosphino)phenyl]ether |
| dba | Dibenzylidenaceton |
| n-BuLi | n-Butyllithium, Lösung in Hexan |
| MTB-Ether | Methyl-tert-butylether |
| THF | Tetrahydrofuran |

Beispiele

Die vorliegende Erfindung wird durch die nachfolgenden nicht einschränkenden Beispiele detailliert beschrieben.

Beispiel 1: 4,6-Difluor-3-trifluormethoxy-7-pentyloxy-dibenzofuran 1.1: 1,2-Difluor-3-pentyloxybenzol

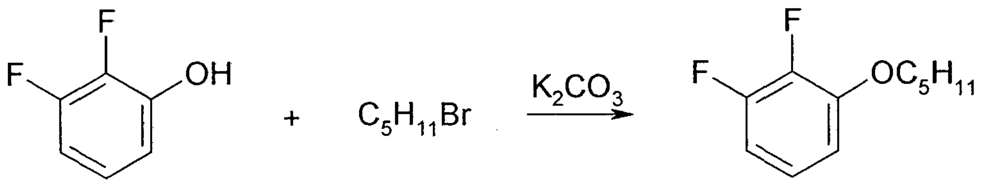


19,0 g (146 mmol) 2,3-Difluorphenol und 20,1 ml (163 mmol) 1-Brompentan werden in Ethylmethylketon gelöst, mit 22,4 g Kaliumcarbonat versetzt und über Nacht zum Sieden erhitzt. Anschließend wird der Feststoff abgetrennt und das Lösungsmittel entfernt. Der erhaltenen Pentyl-2,3-difluorphenylether wird ohne weitere Aufreinigung in der Folgestufe eingesetzt.

1.2: 2,3-Difluor-4-pentyloxybenzolboronsäure

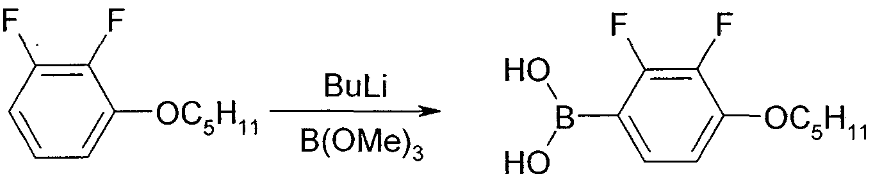


10 11

Eine Lösung von 31.2 g des Rohprodukts aus Stufe 1 (**10**) in 225 ml THF werden bei -70°C mit 108 ml einer 15%igen Lösung von n-Butyllithium in n-Hexan (172 mmol) versetzt. Nach 1h bei dieser Temperatur werden 20 ml (176 mmol) Trimethylborat gelöst in 25 ml THF hinzugefügt. Nach einer weiteren Stunde wird der Ansatz auf 0°C erwärmt, mit Wasser versetzt und mit 25% Salzsäure auf pH 1 eingestellt. Die wässrige Phase wird abgetrennt und mit MTB-Ether extrahiert. Die vereinigten organischen Phasen werden über Natriumsulfat getrocknet und eingeengt. Kristallisation aus n-Heptan liefert 2,3-Difluor-4-pentyloxybenzolboronsäure als farblosen Feststoff.

1.3: 6-Brom-2-fluor-3-trifluormethoxyphenol

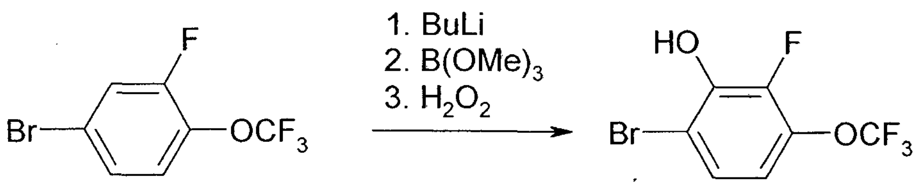


12 13

Unter Stickstoff wird bei einer Temperatur von -70°C eine Lösung von 33 ml (207 mmol) 1-Brom-3-fluor-4-trifluormethoxybenzol (**12**) in 150 ml THF mit 227 mmol einer LDA-Lösung in 100 ml THF, hergestellt aus 32 ml Diisopropylamin und 143 ml einer 15%igen Lösung von n-Butyllithium in n-Hexan, versetzt. Nach 1h bei der Temperatur wird dem Ansatz 26 ml (104 mmol) Trimethylborat, gelöst in 50 ml THF, hinzugefügt. Nach einer weiteren Stunde wird der Ansatz auf 0°C erwärmt, mit 30 ml Eisessig verdünnt mit 38 ml Wasser versetzt. Nach 30 min bei Raumtemp. werden bei eine Temperatur zwischen 35°C und 40°C 45 ml einer 35proz. Wasserstoffperoxidlösung tropfenweise zugesetzt. Nach Beendigung der Zugabe wird der Ansatz 3 h zwischen 35°C und 40°C gehalten, anschließend auf Raumtemp. abgekühlt und mit Wasser und MTB-Ether versetzt. Die organische Phase wird mit Waser und Ammoniumeisen(II)sulfatlösung gewaschen, über Natriumsulfat getrocknet und eingeengt. Der Rückstand wird mit n-Pentan/Dichlormethan über Kieselgel filtriert. Man erhält 6-Brom-2-fluor-3-trifluormethoxyphenol als farbloses Öl.

1.4: 3,2',3'-Trifluor-4'-pentyloxy-4-trifluormethoxy-biphenyl-2-ol

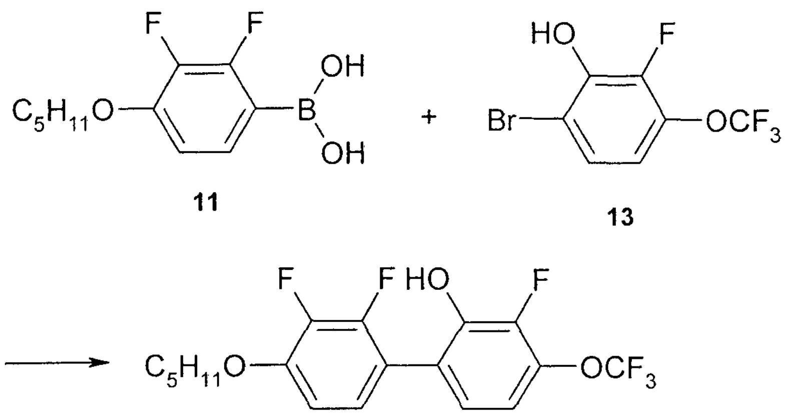

11

13

14

Eine Lösung von 3 g (80%, 9 mmol) 6-Brom-2-fluor-3-trifluormethoxyphenol (**13**) in15 ml THF wird unter Stickstoff mit 1.8 g (13 mmol) Kaliumcarbonat, 6 ml Wasser und bei ca. 60°C mit 40 mg (4 mmol) Tris(dibenzylidenacetone)dipalladium(0) und 50 mg di-(1-adamantyl)butylphosphin versetzt und anschließend zum Sieden erhitzt. In der Siedehitze wird eine Lösung von 2.6 g (97%, 10 mmol) 2,3-Difluor-4-pentyloxybenzolboronsäure (**11**) in 10 ml THF tropfenweise zugegeben. Der Ansatz wird über Nacht unter Rückfluß erhitzt und nach Abkühlen mit Wasser und MTB-Ether versetzt und mit verd. Salzsäure angesäuert. Die wässrige Phase wird mit MTB-Ether extrahiert. Die vereinigten organischen Phasen werden über Natriumsulfat getrocknet, eingeengt und der Rückstand mit Toluol über Kieselgel filtriert. Man erhält 3,2',3'-Trifluor-4'-pentyloxy-4-trifluormethoxy-biphenyl-2-ol als farblosen Feststoff.

1.5: 4,6-Difluor-3-trifluormethoxy-7-pentyloxy-dibenzofuran

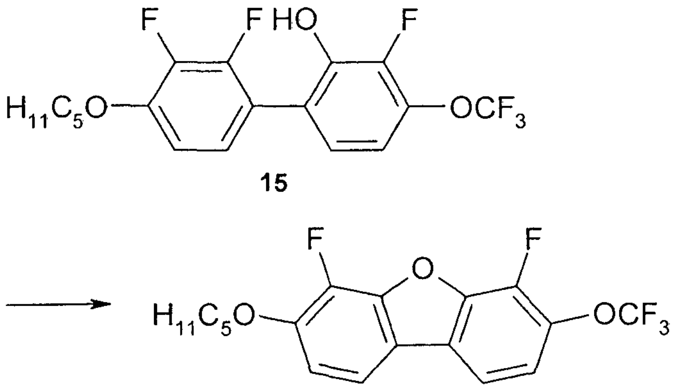

15

16

2,40 g (5,56 mmol) 3,2',3'-Trifluor-4'-pentyloxy-4-trifluormethoxy-biphenyl-2-ol und 15.5 g (6.60 mmol) Kaliumtriphosphat-Monohydrat werden in 20 ml DMPU über Nacht bei 110 °C rühren gelassen. Nach dem Erkalten wird der Ansatz mit MTB-Ether verdünnt, mit Wasser und ges. Kochsalzlösung gewaschen und über Natriumsulfat getrocknet. Das Rohprodukt wird mit Toluol an Kieselgel chromatographiert und aus Toluol umkristallisiert. Man erhält das 4,6-Difluor-3-trifluormethoxy-7-pentyloxy-dibenzofuran als farblose Kristalle vom Schmp. 68 °C.

In Analogie zu Beispiel 1 werden die folgenden Verbindungen hergestellt:

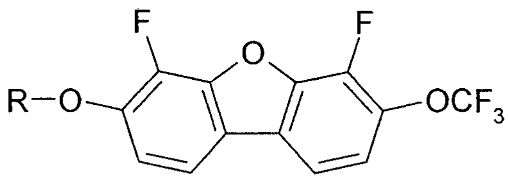

| Bsp. | R | Schmp. [°C] | $\varepsilon_\perp$ | $\Delta\varepsilon$ | $\Delta n$ | $\gamma_1$ [mPa·s] |
|---|---|---|---|---|---|---|
| 1 | n-$C_5H_{11}$ | 68 | 19,81 | -3,55 | 0,1361 | 68 |
| 2 | $CH_3$ | 112 | | | | |
| 3 | $C_2H_5$ | 112 | | | | |
| 4 | n-$C_3H_7$ | 97 | | | | |
| 5 | n-$C_4H_9$ | 87 | | | | |
| 6 | n-$C_6H_{13}$ | 72 | 20,84 | -5,40 | 0,1393 | 100 |

Die aus dem Stand der Technik bekannte Verbindung

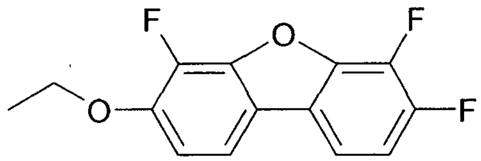

weist folgende Werte auf:

$\Delta\varepsilon$ = -7,79

$\varepsilon_\perp$ = 24,69

$$\varepsilon_\perp / |\Delta\varepsilon| = 3{,}17$$

Für Beispiel 1 erhält man $\varepsilon_\perp / |\Delta\varepsilon|$ = 5,58.

Für Beispiel 6 erhält man $\varepsilon_\perp / |\Delta\varepsilon|$ = 3,86.

Der Vergleich zeigt, dass die erfindungsgemäßen Verbindungen weitaus höhere und damit vorteilhaftere Werte für das Verhältnis von $\varepsilon_\perp$ zu $|\Delta\varepsilon|$ aufweisen.

In Analogie zu Beispiel 1 werden die folgenden Verbindungen hergestellt:

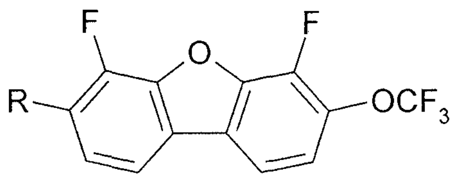

| Bsp. | R | Schmp. [°C] | $\varepsilon_\perp$ | $\Delta\varepsilon$ | $\Delta n$ | $\gamma_1$ [mPa·s] |
|---|---|---|---|---|---|---|
| 7 | $CH_3$ | | | | | |
| 8 | $C_2H_5$ | | | | | |
| 9 | n-$C_3H_7$ | | | | | |
| 10 | n-$C_4H_9$ | | | | | |
| 11 | n-$C_5H_{11}$ | 46 | 16,29 | -3,16 | 0,1113 | 59 |
| 12 | n-$C_6H_{13}$ | | | | | |

In Analogie zu Beispiel 1 werden die folgenden Verbindungen hergestellt:

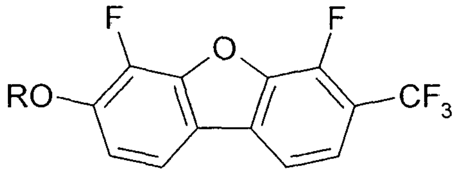

| Bsp. | R | Schmp. [°C] | $\varepsilon_\perp$ | $\Delta\varepsilon$ | $\Delta n$ | $\gamma_1$ [mPa·s] |
|---|---|---|---|---|---|---|
| 13 | $CH_3$ | | | | | |
| 14 | $C_2H_5$ | | | | | |
| 15 | n-$C_3H_7$ | | | | | |
| 16 | n-$C_4H_9$ | | | | | |
| 17 | n-$C_5H_{11}$ | 74 | 23,66 | -2,99 | 0,1333 | 85 |
| 18 | n-$C_6H_{13}$ | 76 | 23,11 | -3,76 | 0,1333 | 89 |

In Analogie zu Beispiel 1 werden die folgenden Verbindungen hergestellt:

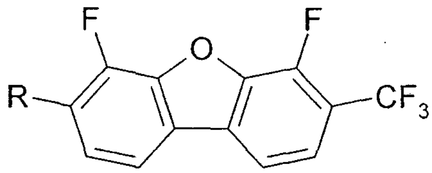

| Bsp. | R | Schmp. [°C] | $\varepsilon_\perp$ | $\Delta\varepsilon$ | $\Delta n$ | $\gamma_1$ [mPa·s] |
|---|---|---|---|---|---|---|
| 19 | $CH_3$ | | | | | |
| 20 | $C_2H_5$ | | | | | |
| 21 | n-$C_3H_7$ | | | | | |
| 22 | n-$C_4H_9$ | | | | | |
| 23 | n-$C_5H_{11}$ | 46 | 18,84 | 2,17 | 0,1212 | 75 |
| 24 | n-$C_6H_{13}$ | | | | | |

Beispiel 25: Butoxy-4,6-difluor-7-trifluormethoxy-dibenzothiophen 25.1: 3,2',3'-Trifluor-4'-butyloxy-4-trifluormethoxy-biphenyl-2-ol

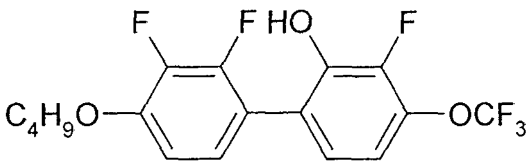

**22**

Phenol **22** wird analog zu Verbindung **14** durch eine Suzuki-Kupplung aus Verbindung **13** und der kommerziell erhältlichen 4-Butoxy-2,3-difluorbenzolboronsäure hergestellt.

25.2: 3,2',3'-Trifluor-4'-butyloxy-4-trifluormethoxy-biphenyl-2-yltrifluormethansulfonsäureester

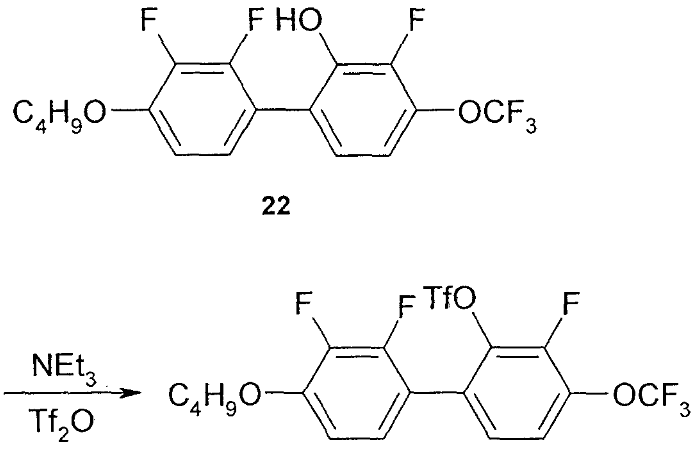


22

23

Eine Lösung von 3,2',3'-Trifluor-4'-butyloxy-4-trifluormethoxy-biphenyl-2-ol (9.7 g) in Dichlormethan (70 ml) wird tropfenweise mit Triethylamin (5.0 ml) und DMAP (60 mg) versetzt. Anschließend wird bei 5 °C Trifluormethansulfonsäureanhydrid (5.0 ml) zugetropft und das Reaktionsgemisch 20 h bei Raumtemperatur gerührt. Es wird über Kieselgel filtriert, mit Dichlormethan gewaschen, getrocknet ($Na_2SO_4$) und im Vakuum eingeengt. Als Rückstand wird der 3,2',3'-Trifluor-4'-butyloxy-4-trifluormethoxy-biphenyl-2-yltrifluormethansulfonsäureester 9 in Form eines schwach gelben Öls isoliert.

25.3: 3-(4'-Butoxy-3,2',3'-trifluor-4-trifluormethoxy-biphenyl-2-ylsulfanyl)-propionsäureethylester

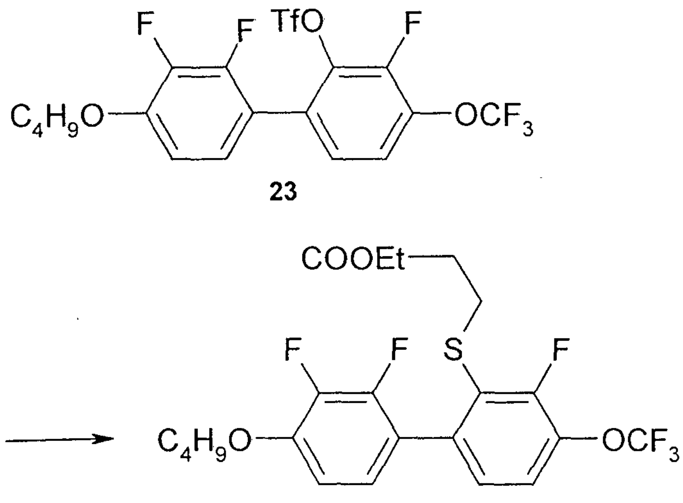

23

24

Eine Lösung aus 3,2',3'-Trifluor-4'-butyloxy-4-trifluormethoxy-biphenyl-2-trifluormethansulfonsäureester (12.7 g) und 3-Mercaptopropionsäureethylester (3.8 ml) in Toluol (55 ml) wird unter Stickstoffatmosphäre zum Rückfluss erhitzt und mit Bis(2-diphenylphosphinophenyl)ether (1.3 g), Tris(dibenzylidenaceton)dipalladium(0) (1.2 g), Kaliumcarbonat (8.4 g) sowie einer kleinen Menge getrocknetem Molekularsieb versetzt. Das Reaktionsgemisch wird 20 h unter Rückfluss erhitzt, anschließend abgekühlt und mit MTB-Ether und dest. Wasser versetzt. Die organische Phase wird abgetrennt, getrocknet ($Na_2SO_4$) und im Vakuum eingeengt. Säulenchromatographische Aufreinigung des Rückstands (Eluent Toluol) liefert 3-(4'-Butoxy-3,2',3'-trifluor-4-trifluormethoxy-biphenyl-2-ylsulfanyl)-propionsäureethylester als gelbes Öl.

25.4: Butoxy-4,6-difluor-7-trifluormethoxy-dibenzothiophen

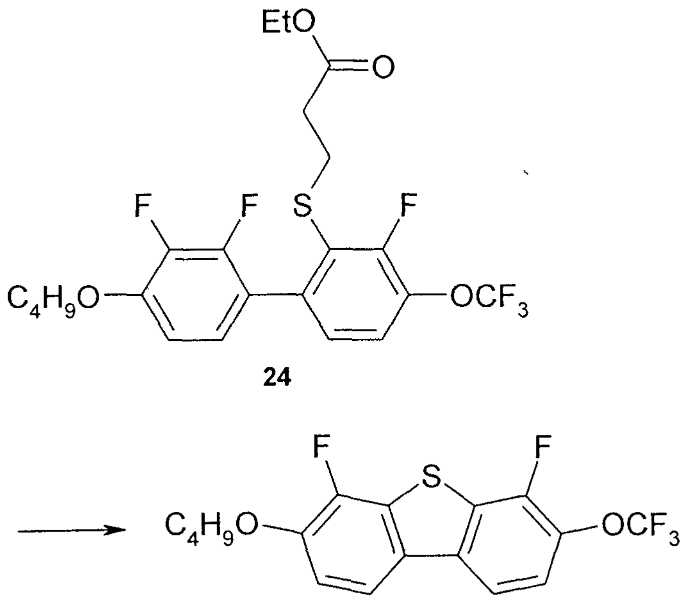

24

25

2,50 g (22,3 mmol) Kalium-tert-butylat werden in 30 ml THF vorgelegt und bei Raumtemp. eine Lösung von 9,20 g (18,3 mmol) 3-(4'-Butoxy-3,2',3'-trifluor-4-trifluormethoxy-biphenyl-2-ylsulfanyl)-propionsäureethylester in 40 ml THF so zutropfen gelassen, dass die Temperatur 30 °C nicht übersteigt. Anschließend wird der Ansatz 1 h unter Rückfluss erhitzt, nach dem Abkühlen mit MTB-Ether verdünnt und mit Wasser gewaschen. Die org. Phase wird über Natriumsulfat getrocknet, das Lösungsmittel i. Vak. entfernt und der Rückstand mit Toluol über Kieselgel filtriert. Kristallisation des Rohprodukts aus Ethanol liefert das 3-Butoxy-4,6-difluor-7-trifluormethoxy-dibenzothiophen als farblose Kristalle vom Schmp. 121 °C.

In Analogie zu Beispiel 25 werden die folgenden Verbindungen hergestellt:

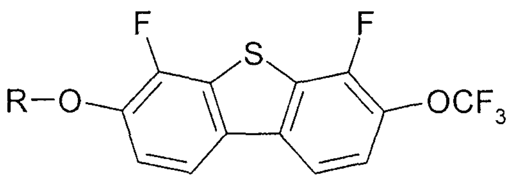

| Bsp. | R | Schmp. [°C] | $\varepsilon_\perp$ | $\Delta\varepsilon$ | $\Delta n$ | $\gamma_1$ [mPa·s] | Phasensequenz |
|---|---|---|---|---|---|---|---|
| 25 | n-$C_4H_9$ | 121 | | | | | |
| 26 | $CH_3$ | | | | | | |
| 27 | $C_2H_5$ | 135 | | | | | |
| 28 | n-$C_3H_7$ | 138 | | | | | |
| 29 | n-$C_5H_{11}$ | 94 | 17,92 | -3,97 | 0,1413 | 103 | K 94 SmA 105 I |
| 30 | n-$C_6H_{13}$ | 114 | | | | | |

In Analogie zu Beispiel 25 werden die folgenden Verbindungen hergestellt:

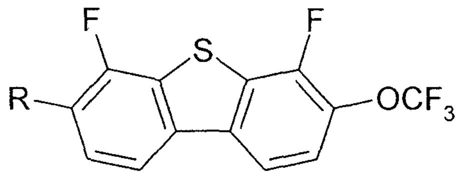

| Bsp. | R | Schmp. [°C] | $\varepsilon_\perp$ | $\Delta\varepsilon$ | $\Delta n$ | $\gamma_1$ [mPa·s] |
|---|---|---|---|---|---|---|
| 31 | $CH_3$ | | | | | |
| 32 | $C_2H_5$ | | | | | |
| 33 | n-$C_3H_7$ | | | | | |
| 34 | n-$C_4H_9$ | | | | | |
| 35 | n-$C_5H_{11}$ | 76 | | | | |
| 36 | n-$C_6H_{13}$ | | | | | |

In Analogie zu Beispiel 25 werden die folgenden Verbindungen hergestellt:

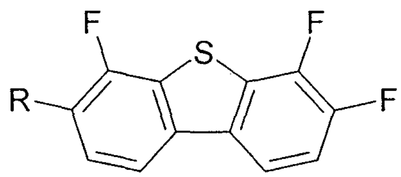

| Bsp. | R | Schmp. [°C] | $\varepsilon_\perp$ | $\Delta\varepsilon$ | $\Delta n$ | $\gamma_1$ [mPa·s] |
|---|---|---|---|---|---|---|
| 37 | $CH_3$ | | | | | |
| 38 | $C_2H_5$ | | | | | |
| 39 | n-$C_3H_7$ | | | | | |
| 40 | n-$C_4H_9$ | | | | | |
| 41 | n-$C_5H_{11}$ | 53 | 19,10 | 5,37 | 0,1293 | 81 |
| 42 | n-$C_6H_{13}$ | | | | | |

In Analogie zu Beispiel 25 werden die folgenden Verbindungen hergestellt:

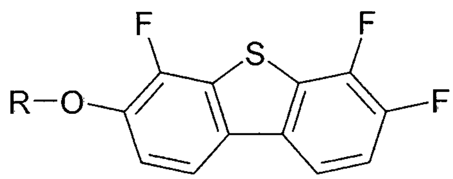

| Bsp. | R | Schmp. [°C] | $\varepsilon_\perp$ | $\Delta\varepsilon$ | $\Delta n$ | $\gamma_1$ [mPa·s] |
|---|---|---|---|---|---|---|
| 43 | $CH_3$ | | | | | |
| 44 | $C_2H_5$ | | | | | |
| 45 | n-$C_3H_7$ | | | | | |

(fortgesetzt)

| Bsp. | R | Schmp. [°C] | $\varepsilon_\perp$ | $\Delta\varepsilon$ | $\Delta n$ | $\gamma_1$ [mPa·s] |
|---|---|---|---|---|---|---|
| 46 | n-$C_4H_9$ | | | | | |
| 47 | n-$C_5H_{11}$ | 75 | | | 0,1492 | 84 |
| 48 | n-$C_6H_{13}$ | | | | | |

In Analogie zu Beispiel 25 werden die folgenden Verbindungen hergestellt:

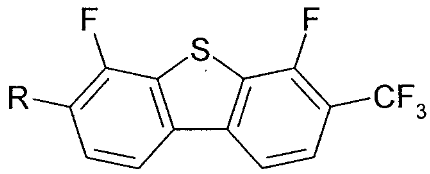

| Bsp. | R | Schmp. [°C] | $\varepsilon_\perp$ | $\Delta\varepsilon$ | $\Delta n$ | $\gamma_1$ [mPa·s] |
|---|---|---|---|---|---|---|
| 49 | $CH_3$ | | | | | |
| 50 | $C_2H_5$ | | | | | |
| 51 | n-$C_3H_7$ | | | | | |
| 52 | n-$C_4H_9$ | | | | | |
| 53 | n-$C_5H_{11}$ | 65 | | | | |
| 54 | n-$C_6H_{13}$ | | | | | |

In Analogie zu Beispiel 25 werden die folgenden Verbindungen hergestellt:

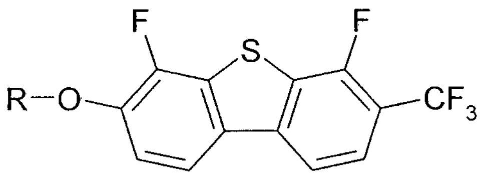

| Bsp. | R | Schmp. [°C] | $\varepsilon_\perp$ | $\Delta\varepsilon$ | $\Delta n$ | $\gamma_1$ [mPa·s] |
|---|---|---|---|---|---|---|
| 55 | $CH_3$ | | | | | |
| 56 | $C_2H_5$ | | | | | |
| 57 | n-$C_3H_7$ | | | | | |
| 58 | n-$C_4H_9$ | | | | | |
| 59 | n-$C_5H_{11}$ | 112 | | | | |
| 60 | n-$C_6H_{13}$ | | | | | |

**Patentansprüche**

**1.** Verbindungen der Formel I

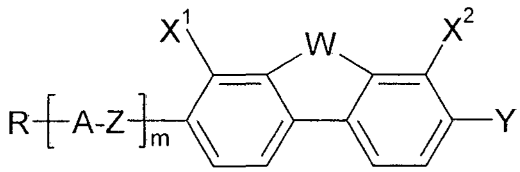

I

worin

W O oder S,
Y F, Cl, $CF_3$, $OCF_3$, $-OCF_2H$, mit der Maßgabe, dass wenn W O bedeutet Y nicht F sein kann,
$X^1$, $X^2$ H oder F, mit der Maßgabe, dass mindestens ein Rest $X^1$ oder $X^2$ F bedeutet,
R einen Alkyl- oder Alkoxyrest mit 1 bis 15 C-Atomen, wobei in diesen Resten auch eine oder mehrere $CH_2$-Gruppen jeweils unabhängig voneinander durch -C≡C-, $-CF_2O-$, $-OCF_2-$, -CH=CH-,

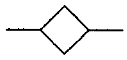 , 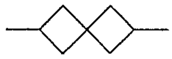 ,

-O-, -CO-O-, oder -O-CO- so ersetzt sein können, dass O-Atome nicht direkt miteinander verknüpft sind, und worin auch ein oder mehrere H-Atome durch Halogen ersetzt sein können,
A bei jedem Auftreten gleich oder verschieden einen Rest ausgewählt aus folgenden Gruppen:

a) 1,4-Phenylen worin auch eine oder zwei CH-Gruppen durch N ersetzt sein können und worin auch ein oder mehrere H-Atome durch eine Gruppe L ersetzt sein können,
b) der Gruppe bestehend aus trans-1,4-Cyclohexylen und 1,4-Cyclohexenylen, worin auch eine oder mehrere nicht benachbarte $CH_2$-Gruppen durch -O- und/oder -S- ersetzt sein können und worin auch ein oder mehrere H-Atome durch F oder Cl ersetzt sein können, und
c) der Gruppe bestehend aus Tetrahydropyran-2,5-diyl, 1,3-Dioxan-2,5-diyl, Tetrahydrofuran-2,5-diyl, Cyclobutan-1,3-diyl, Piperidin-1,4-diyl, Thiophen-2,5-diyl und Selenophen-2,5-diyl, welche auch ein oder mehrfach durch ein Gruppe L substituiert sein können,

L bei jedem Auftreten gleich oder verschieden F, Cl, CN, SCN, $SF_5$ oder geradkettiges oder verzweigtes, jeweils optional fluoriertes, Alkyl, Alkoxy, Alkylcarbonyl, Alkoxycarbonyl, Alkylcarbonyloxy oder Alkoxycarbonyloxy mit 1 bis 12 C-Atomen, und
Z bei jedem Auftreten gleich oder verschieden eine Einfachbindung, $-CF_2O-$, $-OCF_2-$, $-CH_2O-$, $-OCH_2-$, -(CO)O-, -O(CO)-, $-(CH_2)_4-$, $-CH_2CH_2-$ , $-CF_2-CF_2-$ , $-CF_2-CH_2-$, $-CH_2-CF_2-$, -CH=CH- , -CF=CF- , -CF=CH- , -CH=CF-, $-(CH_2)_3O-$, $-O(CH_2)_3-$, -C≡C-, -O-, $-CH_2-$, $-(CH_2)_3-$ oder $-CF_2-$,
m 0, 1 oder 2,

bedeuten.

2. Verbindungen nach Anspruch 1, **dadurch gekennzeichnet, dass** $X^1$ und $X^2$ beide F bedeuten.

3. Verbindungen nach Anspruch 1 oder 2, **dadurch gekennzeichnet, dass** m 0 oder 1 bedeutet.

4. Verbindungen nach einem oder mehreren der Ansprüche 1 bis 3, **dadurch gekennzeichnet, dass** in Formel I Y $CF_3$ oder $OCF_3$ bedeutet.

5. Verbindungen nach Anspruch 4, **dadurch gekennzeichnet, dass** Y $OCF_3$ bedeutet.

6. Verbindungen nach einem oder mehreren der Ansprüche 1 bis 5, **dadurch gekennzeichnet, dass** die Verbindungen der Formel I ausgewählt sind aus den Unterformeln Ia bis Id,

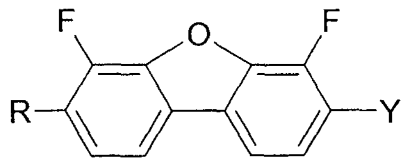

Ia

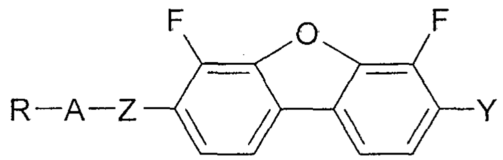

Ib

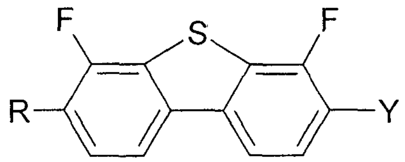

Ic

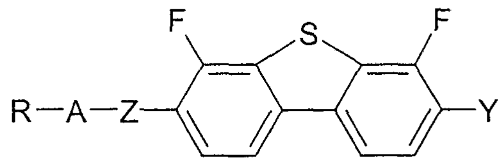

Id

worin R, A, Z und Y die in Anspruch 1 für Formel I angegebenen Bedeutungen haben.

7. Verbindungen nach einem oder mehreren der Ansprüche 1 bis 5, **dadurch gekennzeichnet, dass** W O bedeutet.

8. Verbindungen nach einem oder mehreren der Ansprüche 1 bis 7, **dadurch gekennzeichnet**, das die Verbindungen ausgewählt sind aus den Verbindungen der folgenden Formeln:

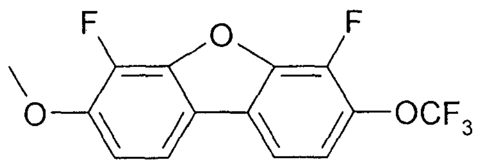

Ia-1-1

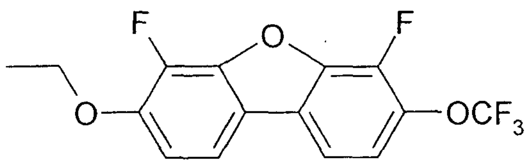

Ia-1-2

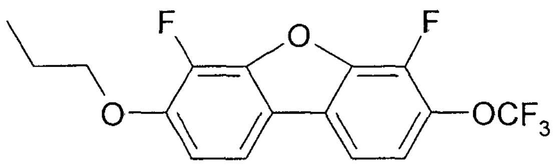

Ia-1-3

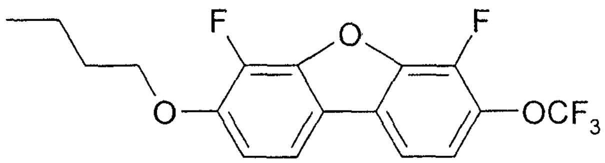
Ia-1-4

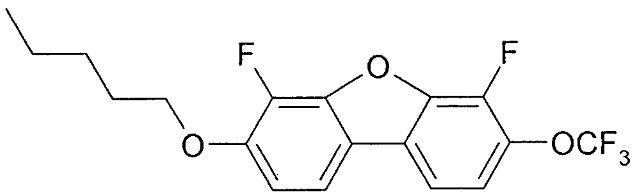
Ia-1-5

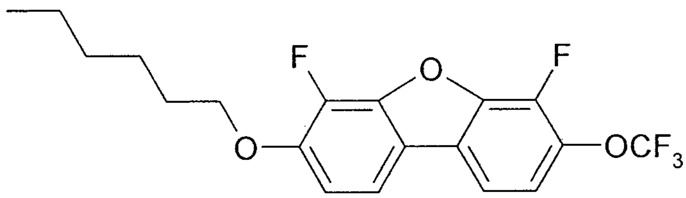
Ia-1-6

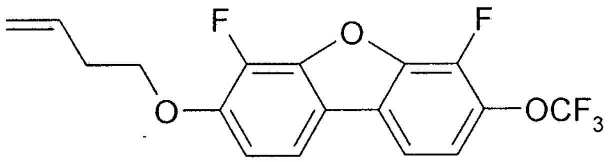
Ia-1-7

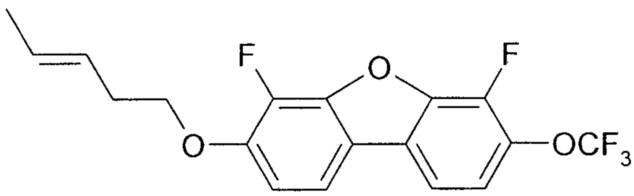
Ia-1-8

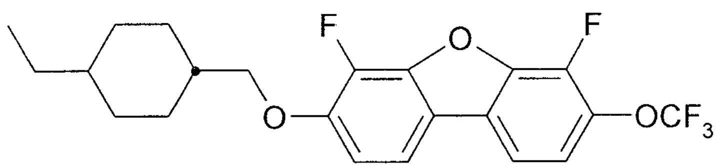
Ib-1-1

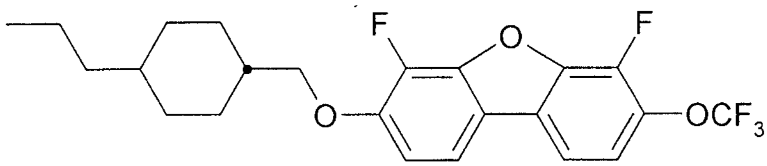
Ib-1-2

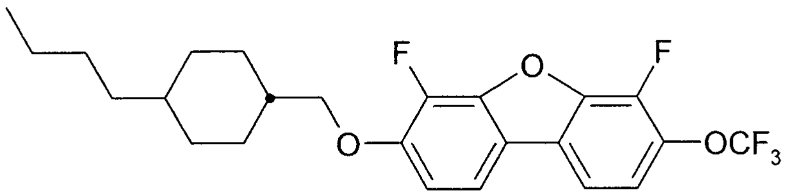

Ib-1-3

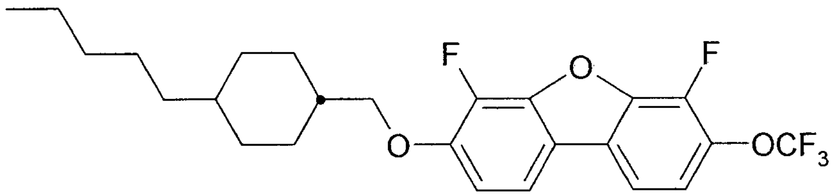

Ib-1-4

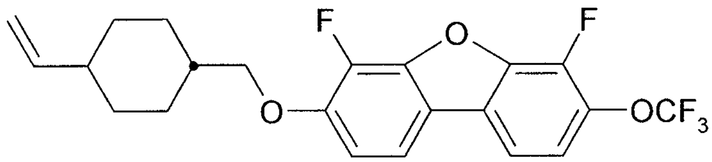

Ib-1-5

**9.** Verwendung einer oder mehrerer Verbindungen nach einem oder mehreren der Ansprüche 1 bis 8 in flüssigkristallinen Medien.

**10.** Flüssigkristallines Medium enthaltend mindestens zwei Verbindungen, **dadurch gekennzeichnet, dass** es mindestens eine Verbindung nach einem oder mehreren der Ansprüche 1 bis 8 enthält.

**11.** Elektrooptisches Anzeigeelement, enthaltend ein flüssigkristallines Medium nach Anspruch 10.

**12.** Verfahren zur Herstellung von Verbindungen der Formel I nach einem oder mehreren der Ansprüche 1 bis 8, **dadurch gekennzeichnet, dass** eine Verbindung der Formel II zu Verbindungen der Formel I umgewandelt wird

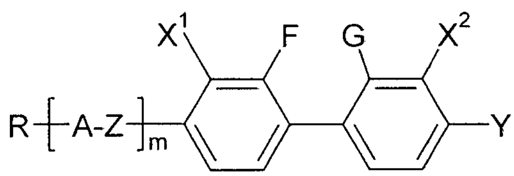

II

wobei R, A, Z, $X^1$, $X^2$, W, Y und m die unter Anspruch 1 angegebene Bedeutung haben und G -OH oder -SG' bedeutet, wobei G' H oder eine Schutzgruppe bedeutet.

## Claims

**1.** Compounds of the formula I

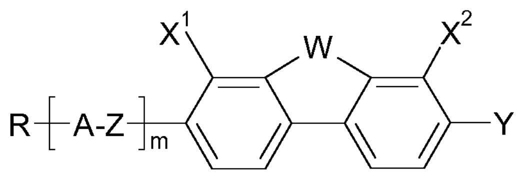

I

In which

W denotes O or S,
Y denotes F, Cl, $CF_3$, $OCF_3$, $OCF_2H$, with the proviso that, if W denotes O, Y cannot be F,
$X^1$, $X^2$ denote H or F, with the proviso that at least one radical $X^1$ or $X^2$ denotes F,
R denotes an alkyl or alkoxy radical having 1 to 15 C atoms, where, in addition, one or more $CH_2$ groups in these radicals may each be replaced, independently of one another, by -C≡C- , $-CF_2O-$, $-OCF_2-$, -CH=CH-,

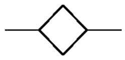 ,

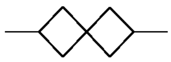 ,

-O-, -CO-O- or -O-CO- in such a way that O atoms are not linked directly to one another, and in which, in addition, one or more H atoms may be replaced by halogen,
A on each occurrence, identically or differently, denotes a radi-cal selected from the following groups:

a) 1,4-phenylene, in which, in addition, one or two CH groups may be replaced by N and in which, in addition, one or more H atoms may be replaced by a group L,
b) the group consisting of trans-1,4-cyclohexylene and 1,4-cyclohexenylene, in which, in addition, one or more non-adjacent $CH_2$ groups may be replaced by -O- and/or -S- and in which, in addition, one or more H atoms may be replaced by F or Cl, and
c) the group consisting of tetrahydropyran-2,5-diyl, 1,3-dioxane-2,5-diyl, tetrahydrofuran-2,5-diyl, cyclobutane-1,3-diyl, piperidine-1,4-diyl, thiophene-2,5-diyl and selenophene-2,5-diyl, each of which may also be mono- or polysubstituted by a group L,

L on each occurrence, identically or differently, denotes F, Cl, CN, SCN, $SF_5$ or straight-chain or branched, in each case optionally fluorinated alkyl, alkoxy, alkylcarbonyl, alkoxy-carbonyl, alkylcarbonyloxy or alkoxycarbonyloxy having 1 to 12 C atoms, and
Z on each occurrence, identically or differently, denotes a single bond, $-CF_2O-$, $-OCF_2-$, $-CH_2O-$, $-OCH_2-$, -(CO)O-, -O(CO)-, $-(CH_2)_4-$, $-CH_2CH_2-$, $-CF_2-CF_2-$, $-CF_2-CH_2-$, $-CH_2-CF_2-$, -CH=CH-, -CF=CF-, -CF=CH-, -CH=CF-, $-(CH_2)_3O-$, $-O(CH_2)_3-$, -C≡C-, -O-, $-CH_2-$, $-(CH_2)_3-$ or $-CF_2-$,
m denotes 0, 1 or 2.

2. Compounds according to Claim 1, **characterised in that** $X^1$ and $X^2$ both denote F.

3. Compounds according to Claim 1 or 2, **characterised in that** m denotes 0 or 1.

4. Compounds according to one or more of Claims 1 to 3, **characterised in that** Y in formula I denotes $CF_3$ or $OCF_3$.

5. Compounds according to Claim 4, **characterised in that** Y denotes $OCF_3$.

6. Compounds according to one or more of Claims 1 to 5, **characterised in that** the compounds of the formula I are selected from the sub-formulae Ia to Id,

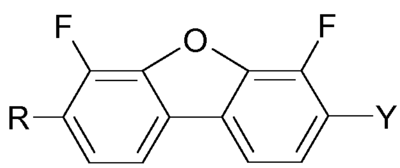

Ia

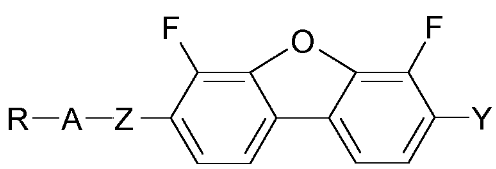

Ib

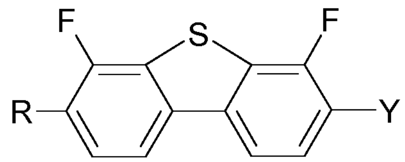

Ic

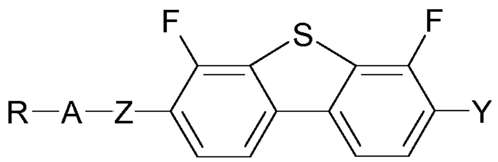

Id

in which R, A, Z and Y have the meanings indicated in Claim 1 for formula I.

7. Compounds according to one or more of Claims 1 to 5, **characterised in that** W denotes O.

8. Compounds according to one or more of Claims 1 to 7, **characterised in that** the compounds are selected from the compounds of the following formulae:

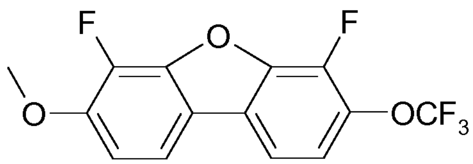

Ia-1-1

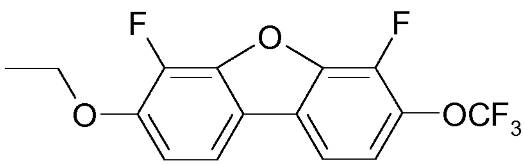

Ia-1-2

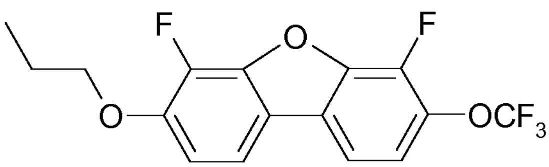

Ia-1-3

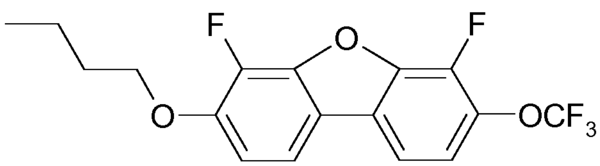

Ia-1-4

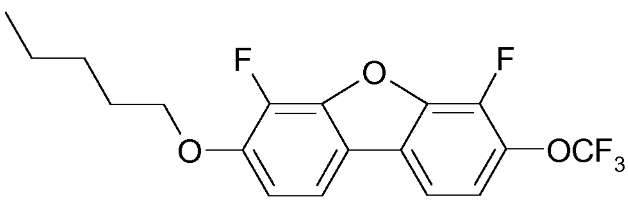

Ia-1-5

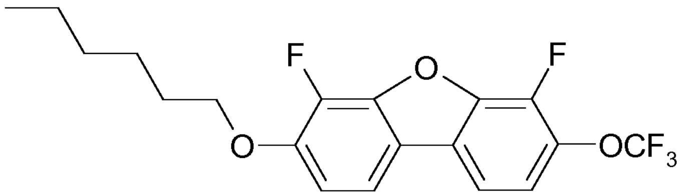

Ia-1-6

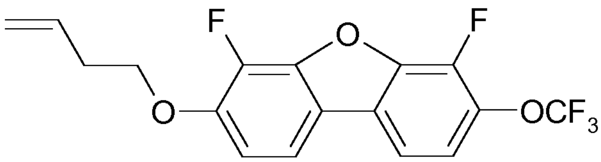

Ia-1-7

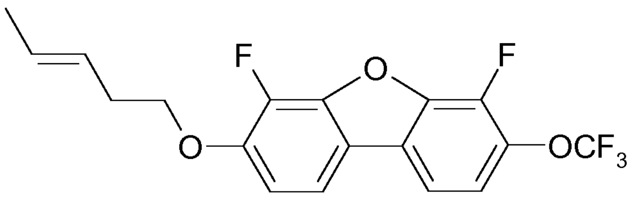

Ia-1-8

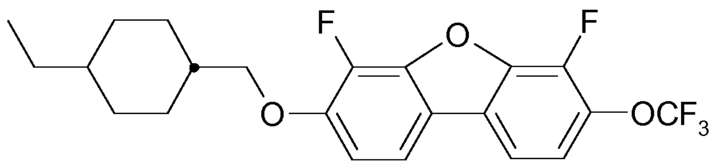

Ib-1-1

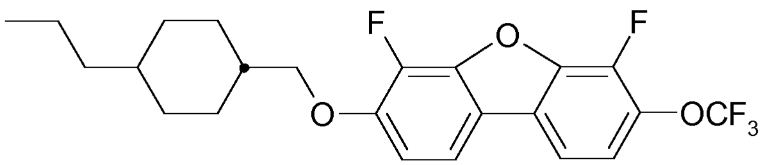

Ib-1-2

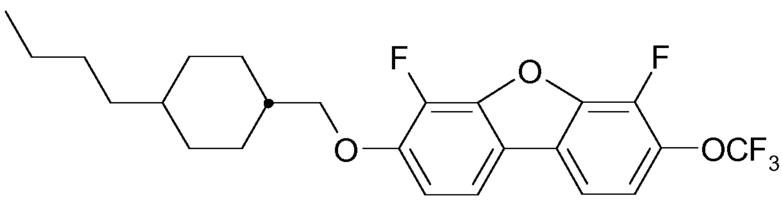

Ib-1-3

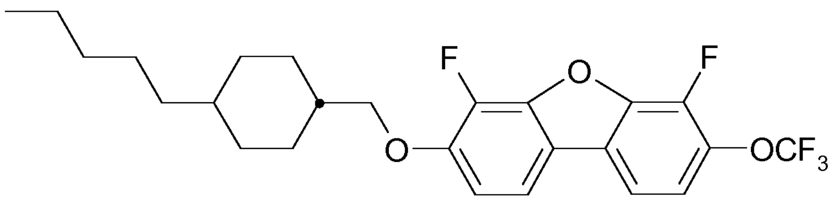

Ib-1-4

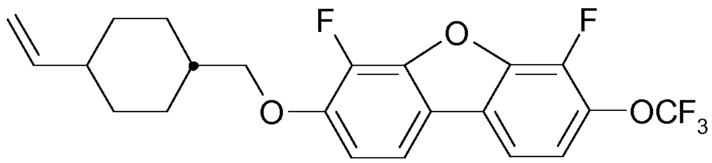

Ib-1-5

**9.** Use of one or more compounds according to one or more of Claims 1 to 8 in liquid-crystalline media.

**10.** Liquid-crystalline medium comprising at least two compounds, **characterised in that** it comprises at least one compound according to one or more of Claims 1 to 8.

**11.** Electro-optical display element containing a liquid-crystalline medium according to Claim 10.

**12.** Process for the preparation of compounds of the formula I according to one or more of Claims 1 to 8, **characterised in that** a compound of the formula II is converted into compounds of the formula I

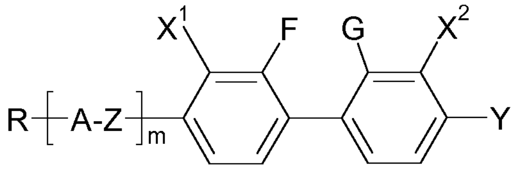

II

where R, A, Z, $X^1$, $X^2$, W, Y and m have the meaning indicated under Claim 1 and G denotes -OH or -SG', where G' denotes H or a protecting group.

## Revendications

**1.** Composés de la formule I :

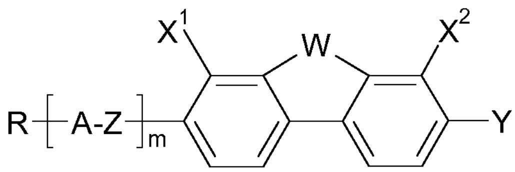

I

dans laquelle :

W représente O ou S ;
Y représente F, Cl, $CF_3$, $OCF_3$, $OCF_2H$, étant entendu que si W représente O, Y ne peut pas être F ;
$X^1$, $X^2$ représente H ou F ; étant entendu qu'au moins un radical $X^1$ ou $X^2$ représente F ;
R représente un radical alkyle ou alcoxy qui comporte de 1 à 15 atome(s) de C, où, en outre, un ou plusieurs groupe(s) $CH_2$ dans ces radicaux peut/peuvent chacun être remplacé(s), de manière indépendante les uns des autres, par -C≡C- , $-CF_2O-$, $-OCF_2-$, -CH=CH-,

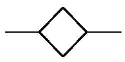

,

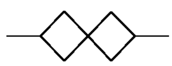

,

-O-, -CO-O- ou -O-CO- de telle sorte que des atomes de O ne soient pas liés directement les uns aux autres, et où, en outre, un ou plusieurs atome(s) de H peut/ peuvent être remplacé(s) par halogène ;
A représente pour chaque occurrence, de manière identique ou différente, un radical qui est sélectionné parmi les groupes qui suivent :

a) 1,4-phénylène, où, en outre, un ou deux groupe(s) CH peut/peuvent être remplacé(s) par N et où, en outre, un ou plusieurs atome(s) de H peut/peuvent être remplacé(s) par L ;

b) le groupe qui est constitué par trans-1,4-cyclohexylène est 1,4-cyclohexénylène, où, en outre, un ou plusieurs groupe(s) $CH_2$ non adjacents peut/peuvent être remplacé(s) par -O- et/ou par -S- et où, en outre, un ou plusieurs atome(s) de H peut/peuvent être remplacé(s) par F ou par Cl ; et

c) le groupe qui est constitué par tétrahydropyran-2,5-diyle, 1,3-dioxane-2,5-diyle, tétrahydrofuran-2,5-diyle, cyclobutane-1,3-diyle, pipéridine-1,4-diyle, thiophène-2,5-diyle et sélénophène-2,5-diyle, dont chacun peut également être mono- ou polysubstitué par un groupe L ;

L représente pour chaque occurrence, de manière identique ou différente, F, Cl, CN, SCN, $SF_5$ ou alkyle, alcoxy, alkyl-carbonyle, alcoxycarbonyle, alkylcarbonyloxy ou alcoxy-carbonyloxy en chaîne droite ou ramifié, dans chaque cas en option fluoré, qui comporte de 1 à 12 atome(s) de C ; et

Z représente pour chaque occurrence, de manière identique ou différente, une liaison simple, $-CF_2O-$, $-OCF_2-$, $-CH_2O-$, $-OCH_2-$, -(CO)O-, -O(CO)-, $-(CH_2)_4-$, $-CH_2CH_2-$, $-CF_2-CF_2-$, $-CF_2-CH_2-$, $-CH_2-CF_2-$, -CH=CH-, -CF=CF-, -CF=CH-, -CH=CF-, $-(CH_2)_3O-$, $-O(CH_2)_3-$, -C≡C-, -O-, $-CH_2-$, $-(CH_2)_3-O$ u $-CF_2-$ ;

m représente 0, 1 ou 2.

2. Composés selon la revendication 1, **caractérisés en ce que** $X^1$ et $X^2$ représentent tous deux F.

3. Composés selon la revendication 1 ou 2, **caractérisés en ce que** m représente 0 ou 1.

4. Composés selon une ou plusieurs des revendications 1 à 3, **caractérisés en ce que** Y dans la formule I représente $CF_3$ ou $OCF_3$.

5. Composés selon la revendication 4, **caractérisés en ce que** Y représente $OCF_3$.

6. Composés selon une ou plusieurs des revendications 1 à 5, **caractérisés en ce que** les composés de la formule I sont sélectionnés parmi les sous-formules Ia à Id :

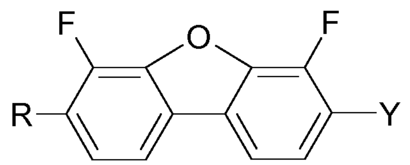

Ia

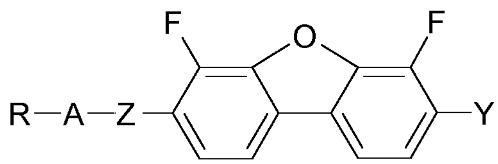

Ib

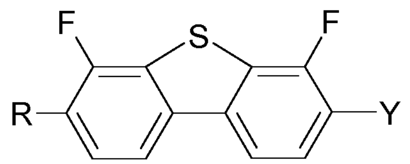

Ic

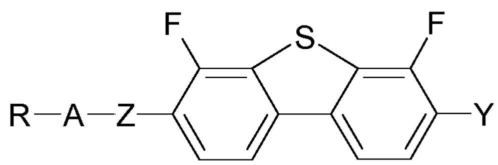

Id

dans lesquelles R, A, Z et Y présentent les significations qui ont été indiquées selon la revendication 1 pour la formule I.

**7.** Composés selon une ou plusieurs des revendications 1 à 5, **caractérisés en ce que** W représente O.

**8.** Composés selon une ou plusieurs des revendications 1 à 7, **caractérisés en ce que** les composés sont sélectionnés parmi les composés des formules qui suivent :

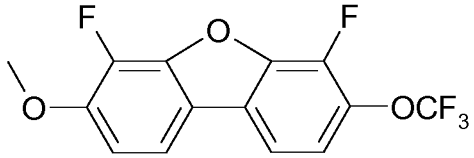

Ia-1-1

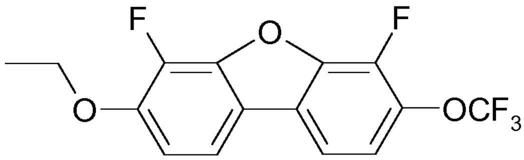

Ia-1-2

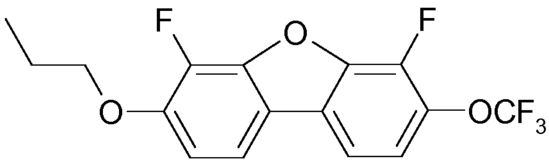

Ia-1-3

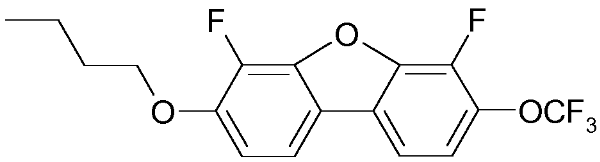

Ia-1-4

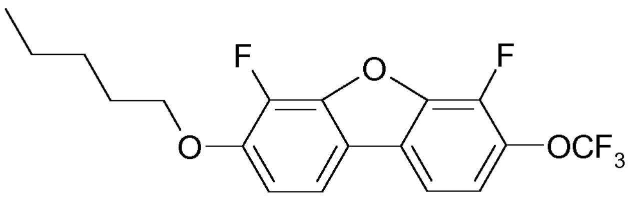

Ia-1-5

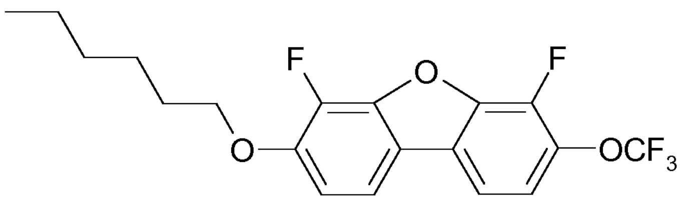

Ia-1-6

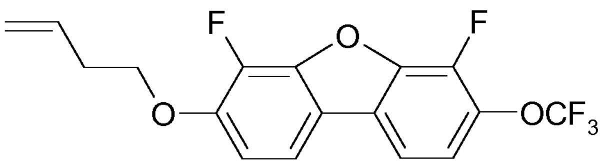

Ia-1-7

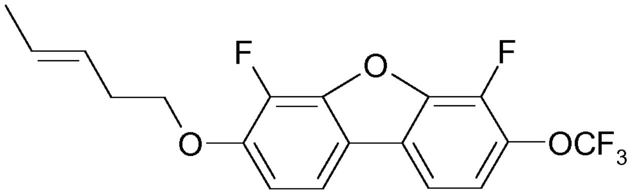

Ia-1-8

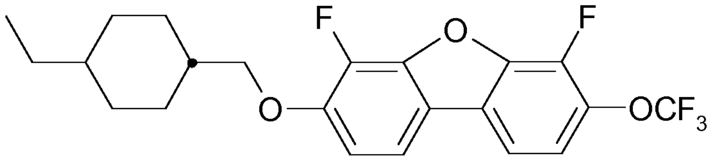

Ib-1-1

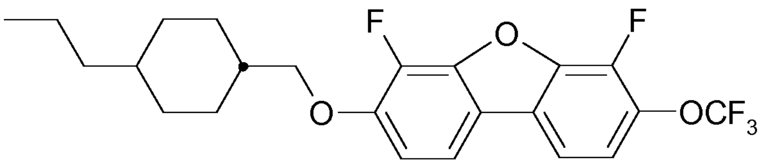

Ib-1-2

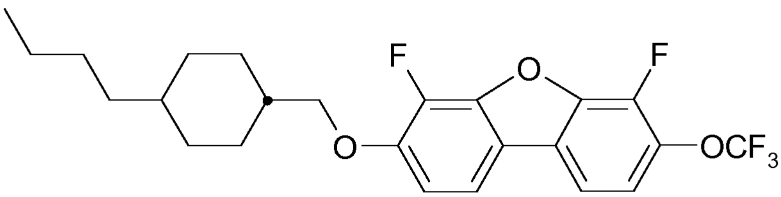

Ib-1-3

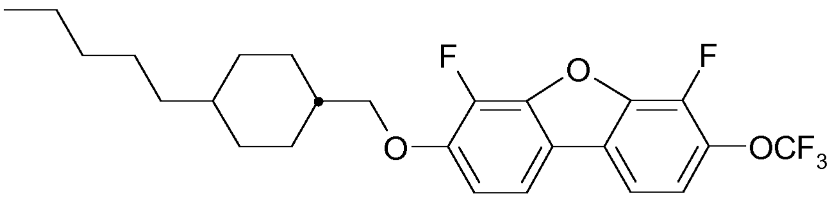

Ib-1-4

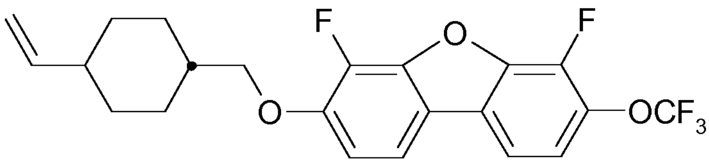

Ib-1-5

**9.** Utilisation d'un ou de plusieurs composé(s) selon une ou plusieurs des revendications 1 à 8 dans des milieux cristallins liquides.

**10.** Milieu cristallin liquide comprenant au moins deux composés, **caractérisé en ce qu'**il comprend au moins un composé selon une ou plusieurs des revendications 1 à 8.

**11.** Élément d'affichage électro-optique contenant un milieu cristallin liquide selon la revendication 10.

**12.** Procédé pour la préparation de composés de la formule I selon une ou plusieurs des revendications 1 à 8, **caractérisé en ce qu'**un composé de la formule II est converti selon des composés de la formule I

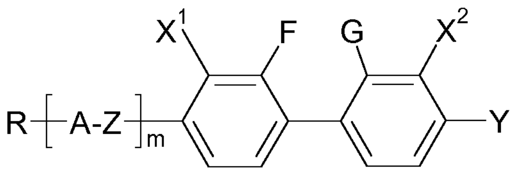

II

dans laquelle R, A, Z, $X^1$, $X^2$, W, Y et m présentent la signification qui a été indiquée selon la revendication 1 et G représente -OH ou -SG', où G' représente H ou un groupe de protection.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2628779 A2
- WO 02055463 A
- DE 102004021691 A1
- DE 102005012585 A1


### In der Beschreibung aufgeführte Nicht-Patentliteratur


- **H. KELKER/R. HATZ.** Handbook of Liquid Crystals. Verlag Chemie, 1980
- Merck Liquid Crystals - Licristal® - Physical Properties of Liquid Crystals - Description of the Measurements Methods. *Merck KGaA,* 1998